(19) Europäisches Patentamt
European Patent Office
Office européen des brevets

(11) EP 0 498 331 B1

(12) EUROPEAN PATENT SPECIFICATION

(45) Date of publication and mention of the grant of the patent:
**22.05.1996 Bulletin 1996/21**

(51) Int Cl.[6]: **C07D 451/02**, A61K 31/46

(21) Application number: **92101706.7**

(22) Date of filing: **03.02.1992**

# (54) N-(aryloxyalkyl)heteroaryl-8-azabicyclo(3.2.1)octanes, intermediates and a process for the preparation thereof and their use as medicaments

N-(Aryloxyalkyl)heteroaryl-8-Azabicyclo[3.2.1]Octan, Zwischenverbindungen und Verfahren zu deren Herstellung und deren Verwendung als Arzneimittel

N-(aryloxyalkyl)hétéroaryl-aza-8-bicyclo[3.2.1]octanes, des intermédiaires et un procédé pour leur préparation et leur utilisation comme médicaments

(84) Designated Contracting States:
**AT BE CH DE DK ES FR GB GR IT LI LU NL PT SE**

(30) Priority: **04.02.1991 US 650144**

(43) Date of publication of application:
**12.08.1992 Bulletin 1992/33**

(73) Proprietor: **HOECHST-ROUSSEL PHARMACEUTICALS INCORPORATED**
**Somerville, NJ 08876-1258 (US)**

(72) Inventors:
- **Glamkowski, Edward J.**
  **Warren, NJ 07060 (US)**
- **Fink, David M.**
  **Doylestown, PA 18901 (US)**
- **Kurys, Barbara E.**
  **Elmwood Park, NJ 07407 (US)**

(74) Representative: **Ackermann, Joachim et al**
**Cohausenstrasse 1**
**D-65719 Hofheim/Ts (DE)**

(56) References cited:
**EP-A- 0 315 390** **FR-A- 2 548 666**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 498 331 B1

Printed by Jouve, 75001 PARIS (FR)

## Description

The present invention relates to N-(aryloxyalkyl)heteroaryl-8-azabicyclo[3.2.1]octanes, intermediates, a process for the preparation thereof and their use as medicaments.

FR 2 548 666 discloses compounds which are stated to be useful as antipsychotic agents of the formula:

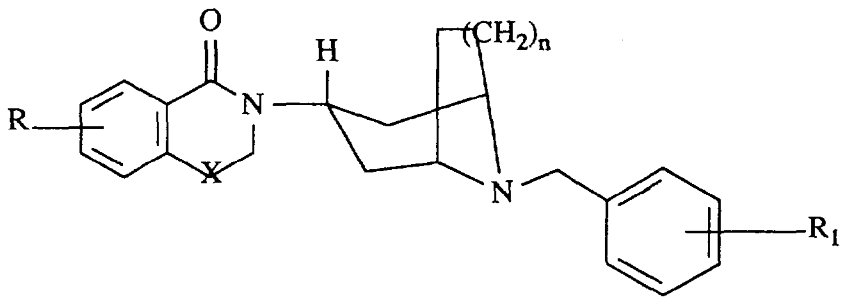

where

X is $CH_2$, $CH_2$-$CH_2$ or O-$CH_2$,
n is 1 or 2,
R is H, halogen, $CH_3$ or $CH_3O$, and
$R_1$ is H, $CH_3$, $CH_3O$, CN or one or two halogen atoms.

The present invention provides to compounds of the formula

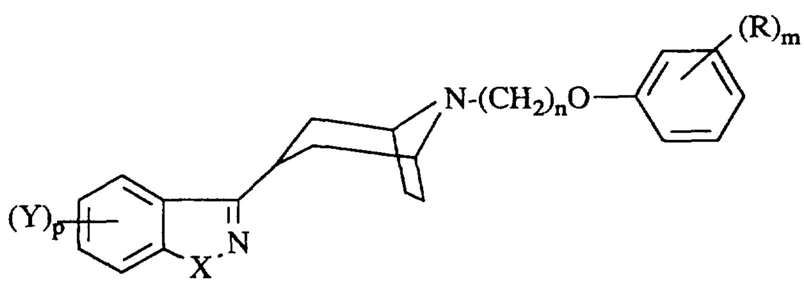

(I)

where

X is -O-, -S- or -NH-;
Y is hydrogen, halogen and loweralkoxy;
p is 1 or 2;
n is 2, 3 or 4;
R is hydrogen, loweralkyl, loweralkoxy, hydroxy, halogen, amino, loweralkylamino, nitro, loweralkylthio, trifluoromethoxy, cyano, trifluoromethyl,

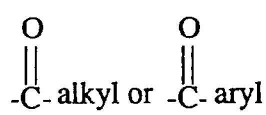

where aryl is

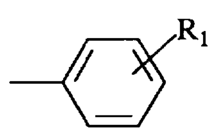

;

$R_1$ is hydrogen, loweralkyl, loweralkoxy, halogen, hydroxy, carboxyl, loweralkylamino, nitro, loweralkylthio, cyano, and trifluoromethyl;
m is 1 or 2; or a compound of the above formula where R is 3-methoxy and 1-acetamide, n is 3, X is -O- and Y is 6-fluoro; a pharmaceutically acceptable acid addition salt thereof, and, where applicable the geometric and optical isomers and racemic mixtures thereof.

The compounds and compositions of this invention are useful as antipsychotic agents and as inhibitors of the reuptake of serotonin.

Subgeneric to the compounds of formula I are compounds of formula II

(II)

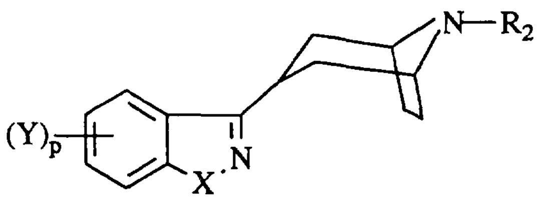

wherein $R_2$ is hydrogen or loweralkyl and X, Y, and p are as previously defined.

This invention also relates to compounds of formula (III)

(III)

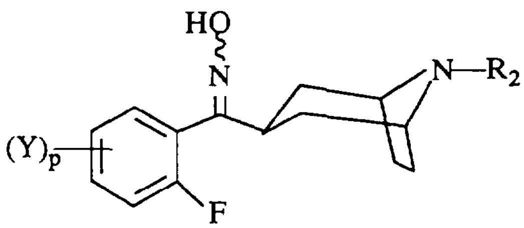

where Y, $R_2$, and p are as defined above, which are useful as intermediates for the preparation of compounds of formula II.

Additionally, this invention relates to compounds of the formula IV

(IV)

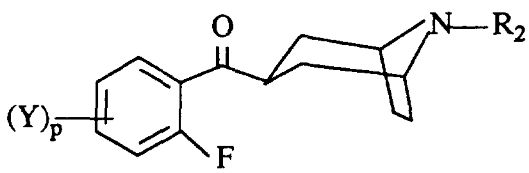

where $R_2$, Y are p are as previously defined which are useful as intermediates for the preparation of compounds of formula II and III.

Preferred embodiments of the invention are those of formula I wherein X is O or S, n is 3 and R is $OCH_3$ and

$$-\overset{\overset{O}{\|}}{C}-$$

alkyl wherein m=2.

Most preferred embodiments of the invention are those of formula I wherein X is O, n is 3 and R is $OCH_3$ and

$$-\overset{\overset{O}{\|}}{C}-$$

$CH_3$ where in m=2.

Throughout the specification and appended claims, a given chemical formula or name shall encompass all geometric and optical isomers and racemic mixtures where such isomers and mixtures exist, as well as pharmaceutically acceptable acid addition salts thereof and solvates thereof such as hydrate.

In the above definitions, the term "lower" means that the group it is describing contains from 1 to 6 carbon atoms.

The term "alkyl" refers to a straight or branched chain hydrocarbon containing no unsaturation, for example, methyl, ethyl, isopropyl, 2-butyl, neopentyl, n-hexyl, etc.

The term "alkoxy" refers to a monovalent substituent comprising an alkyl group linked through an ether oxygen having its free valence bond from the ether oxygen, e.g. methoxy, ethoxy, propoxy, butoxy, pentoxy, etc.

The term "aryl" means

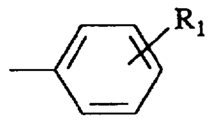

wherein $R_1$ is hydrogen, loweralkyl, loweralkoxy, hydroxy, halogen, loweralkylamino, nitro, cyano or trifluoromethyl.

The term "loweralkylthio" refers to a monovalent substituent having the formula loweralkyl-S-.

The term "halogen" refers to a member of the halogen family consisting of fluorine, chlorine, bromine and iodine.

The compounds of this invention are prepared in the following manner. The substituents are as defined above unless indicated otherwise.

An aldehyde of the formula

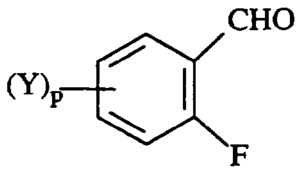

is reacted with an orthoformate in the presence of a catalytic amount of p-toluenesulfonic acid to afford compound (V) of the formula

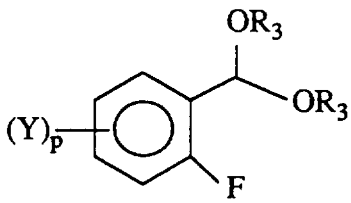

(V)

where $R_3$ is loweralkyl. This reaction typically takes place in a loweralkanol solvent such as ethanol, methanol, etc. at a temperature of 0 to 50°C for 10 to 24 hours.

Compound V is subsequently reacted with a phosphorylating agent such as triethyl phosphite and with boron trifluoride etherate in a suitable solvent such as dichloromethane to afford Compound VI of the formula

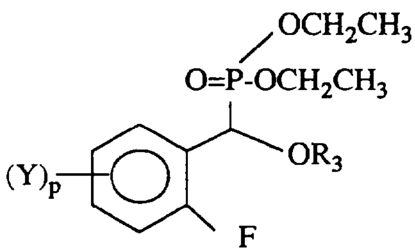

(VI)

This reaction typically takes place at a temperature of -25°C to room temperature for 10 to 30 hours.

Compound VI is reacted with n-butyllithium or other suitable agents such as lithium diisopropyl amide and tropinone of the formula

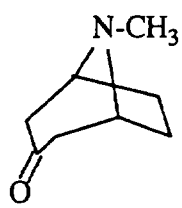

to afford Compound VII of the formula

(VII)

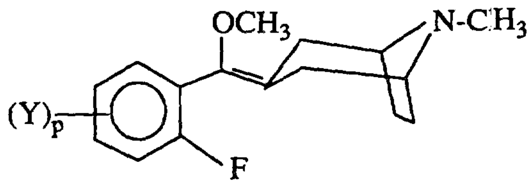

Typically, this reaction takes place in a suitable solvent such as tetrahydrofuran at a temperature of -78°C to room temperature for 10 to 30 hours.

Compound VII is reacted with aqueous HCl in acetone at reflux for 2 to 8 hours to afford Compound VIII of the invention of the formula

(VIII)

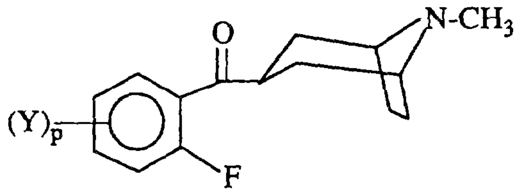

Compound VIII is the intermediate which is generally used for the preparation of the target compounds of this invention.

Preparation of Compounds where X is -O-.

Compounds of the formula

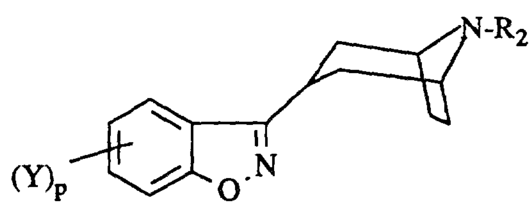

for use in synthesizing the benzisoxazole-substituted compounds of the invention can be prepared as follows.

Compound VIII is reacted with hydroxylamine hydrochloride and ammonium acetate to afford Compound IX of the invention of the formula

(IX)

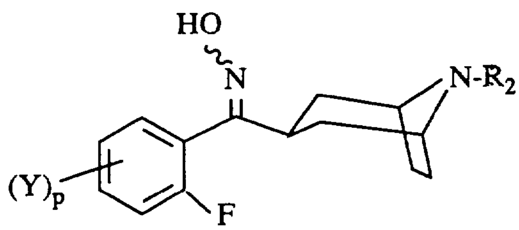

This reaction typically takes place at reflux for 3 to 25 hours in the presence of a solvent such as aqueous ethanol, methanol, etc.

Compound IX is cyclized using a base such as sodium hydroxide or sodium ethoxide heated at reflux to afford Compound X of the invention of the formula

(X)

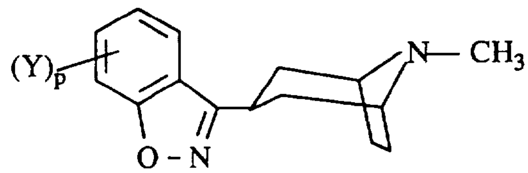

This reaction is conducted in a suitable solvent such as ethanol, methanol etc. for 2 to 10 hours.

Compound X can undergo cleavage of the methyl group by reacting it with vinyl chloroformate or other suitable demethylating agent and then heating to reflux in the presence of a strong acid to afford Compound XI of the invention of the formula

(XI)

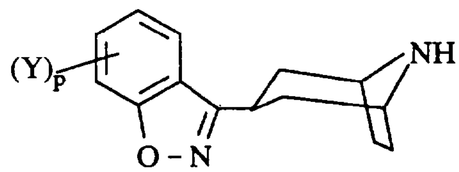

The reaction with vinyl chloroformate takes place in a halogenated hydrocarbon solvent such as 1,2-dichloroethane or chloroform for 2 to 10 hours. The reaction with a strong acid such as HCl also takes place at reflux temperature in a suitable solvent such as ethanol for 1 to 5 hours.

Compound XI is reacted with potassium carbonate and Compound XII of the general formula

(XII)

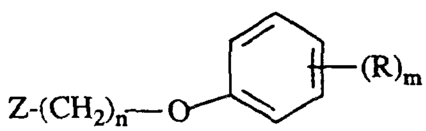

where Z is chlorine or bromine and n is 2, 3 or 4 to afford the target benzisoxazoles of the invention of the formula

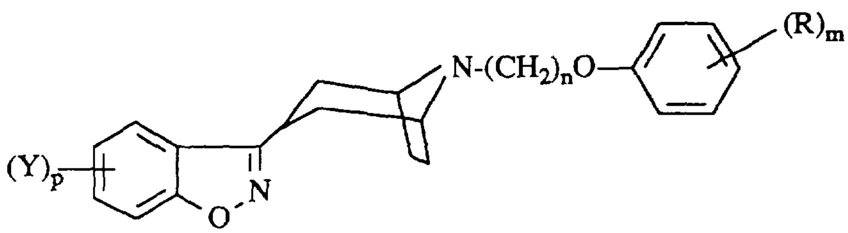

(Ia)

This reaction generally takes place in a suitable solvent such as acetonitrile, dimethylformamide, etc. optionally using a catalytic amount of potassium iodide at reflux for 10 to 30 hours.

Preparation of Compounds Where X is

-N-
H

Compounds of the formula

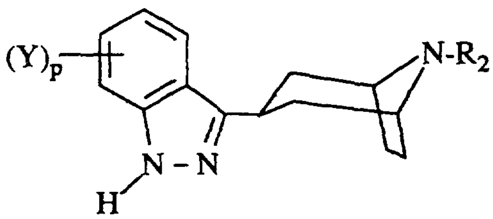

for use in synthesizing the indazoyl-substituted compounds of the invention can be prepared as follows.

Compound VIII is reacted with hydrazine hydrate under standard conditions in a suitable solvent such as ethanol or methanol to afford Compound XIII of the formula

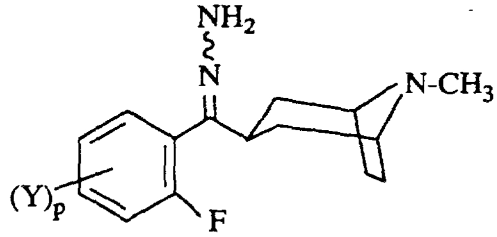

(XIII)

This reaction typically takes place at reflux for 4 to 20 hours.

Compound XIII is cyclized by reacting the hydrazone and potassium carbonate in a suitable solvent such as dimethylformamide to afford Compound XIV of the invention of the formula

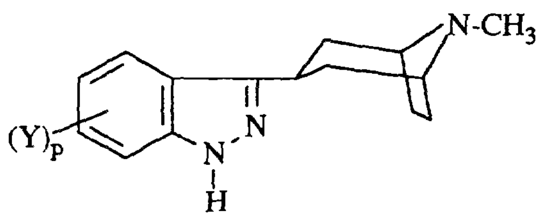

(XIV)

Typically, the reaction is carried out at 90° to reflux for 4 to 20 hours.

Compound XIV is reacted with potassium carbonate and cyanogen bromide to form intermediate XV of the formula

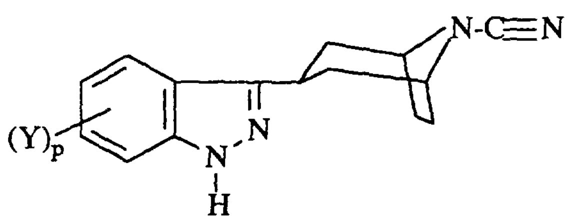

(XV)

This reaction is carried out in a dipolar aprotic solvent such as dimethylformamide or dimethylsulfoxide at ambient temperatures for 2 to 20 hours.

Compound XV is subsequently reduced by means of a metal hydride, e.g., with lithium aluminum hydride to afford Compound XVI of the invention of the formula

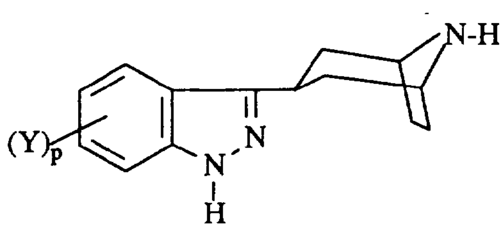

(XVI)

Typically this reaction is conducted in an ethereal solvent such as tetrahydrofuran or diethylether at reflux for 1 to 5 hours.

Compound XVI, the immediate precursor of the target indazoles, is reacted with Compound XII to afford the target

indazoles of the invention of the formula

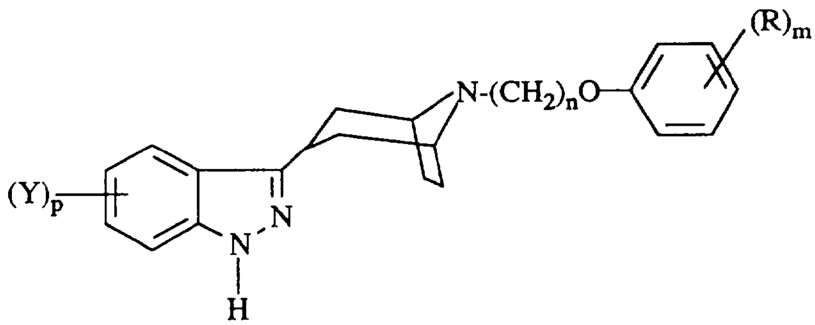

This reaction takes place under essentially the same conditions as the synthesis of the benzisoxazole substituted target compounds of the invention.

Preparation of Compounds Where X is -S-.

Compounds of the formula

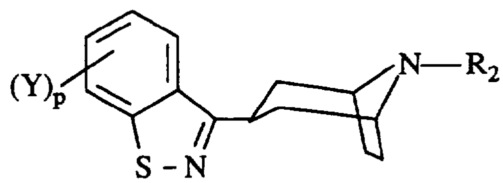

for use in the synthesis of benzisothiazolyl compounds of the invention can be prepared as follows.

Compound VIII is reacted with sulphur in a solution of ammonia in a solvent such as 2-methoxyethanol to afford Compound XVII of the invention of the formula

(XVII)

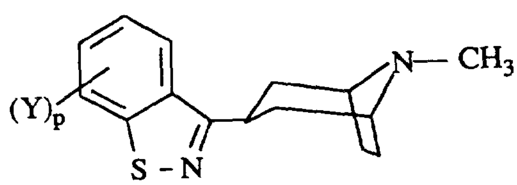

Typically, this reaction takes place at a temperature of about 100 to 180°C for 2 to 5 hours.

Compound XVII is subsequently reacted with vinyl chloroformate and potassium carbonate at reflux in a suitable solvent such as dichloroethane for 2 to 10 hours and the resultant product is further reacted with HCl in ethanol at reflux in a standard cleavage reaction to form Compound XVIII of the invention of the formula

(XVIII)

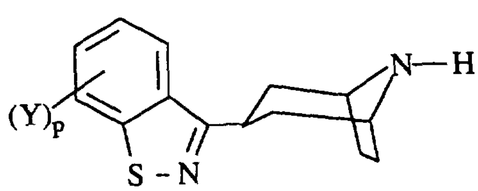

Compound XVIII, the immediate precursor of the target benzisothiazoles is alkylated in a manner similar to the alkylation of the other target compounds of the invention.

The compounds of the present invention are useful for treating psychoses by virtue of their ability to elicit an antipsychotic response in animals. Antipsychotic activity is determined in the climbing mice assay by a method similar to to those described by P. Protais, et al., Psychopharmacol., 50:1(1976) and B. Costall, Eur. J. Pharmacol., 50:39 (1978).

Subject CK-1 male mice (23-27 grams) are grouped-housed under standard laboratory conditions. The mice are individually placed in wire mesh stick cages (4" X 10") and are allowed one hour for adaption and exploration of the

new environment. Then apomorphine is injected subcutaneously at 1.5 mg/kg, a dose causing climbing in all subjects for 30 minutes. Compounds to be tested for antipsychotic activity are injected intraperitoneally or given oral doses at various time intervals, e.g. 30 minutes, 60 minutes, etc. prior to the apomorphine challenge at a screening dose of 10-60 mg/kg.

For evaluation of climbing, 3 readings are taken at 10, 20 and 30 minutes after apomorphine administration according to the following scale:

| Climbing Behavior Mice with: | Score |
|---|---|
| 4 paws on bottom (no climbing) | 0 |
| 2 paws on the wall (rearing) | 1 |
| 4 paws on the wall (full climb) | 2 |

Mice consistently climbing before the injection of apomorphine are discarded.

With fully-developed apomorphine climbing, the animals are hanging on to the cage walls, rather motionless, over longer periods of time. By contrast, climbs due to mere motor stimulation usually only last a few seconds.

The climbing scores are individually totaled (maximal score: 6 per mouse over 3 readings) and the total score of the control group (vehicle intraperitoneally-apomorphine subcutaneously) is set to 100%. $ED_{50}$ values with 95% confidence limits, calculated by a linear regression analysis, of representative compounds of the present invention as well as a standard antipsychotic agent are presented in Table 1.

TABLE 1

| COMPOUND | CLIMBING MOUSE ASSAY ($ED_{50}$ mg/kg, ip) |
|---|---|
| [4-[3-[3-[6-Fluoro-1,2-benzisoxazol-3-yl]-8-azabicyclo[3.2.1] octan-8-yl]propoxy]-3-methoxyphenyl] ethanone monohydrochloride | 5.0 |
| [4-[2-[3-[6-Fluoro-1,2-benzisoxazol-3-yl]-8-azabicyclo[3.2.1] octan-8-yl]ethoxy]-3-methoxyphenyl]ethanone fumarate | 12.5 |
| Clozapine (reference) | 8.1 |

Antipsychotic response is achieved when the compounds of the present invention are administered to a subject requiring such treatment as an effective oral, parenteral, or intravenous dose of from 0.01 to 50 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and they do not, to any extent, limit the scope or practice of the invention.

The compounds of the present invention may also be useful for the treatment of depression and/or obsessive-compulsive disorder by virtue of their ability to inhibit the reuptake of serotonin.

Some researchers have suggested that subjects with serotonergic hypofunction comprise a biochemical subgroup of depressed patients. Others claim that altered serotonergic function determines the change associated with obsessive-compulsive disorder.

This activity is determined in an assay which measures $^3H$-serotonin uptake in rat whole brain and hypothalamic synaptosomes. The assay described below is used as a biochemical screen for potential antidepressants which block serotonin (5-hydroxytryptamine [5HT]) uptake.

[$^3H$]-5HT transport has been characterized in the central nervous system tissue and found to be saturable, sodium and temperature-dependent, inhibited by ouabain, metabolic inhibitors, tryptamine analogs and tricyclic antidepressents.

Procedure

A. Animals

Male CR Wistar rats

B. Reagents

1. Krebs-Henseleit Bicarbonate Buffer, pH 7.4 (KHBB):

Prepare a 1 liter batch containing the following salts.

| | grams/l | mM |
|---|---|---|
| NaCl | 6.92 | 118.4 |
| KCl | 0.35 | 4.7 |
| $MgSO_4 \cdot 7H_2O$ | 0.29 | 1.2 |
| $KH_2PO_4$ | 0.16 | 2.2 |
| $NaHCO_3$ | 2.10 | 24.9 |
| $CaCl_2$ | 0.14 | 1.3 |

Prior to use add:

| | | |
|---|---|---|
| Dextrose | 2 mg/ml | 11.1 |
| Iproniazid phosphate | 0.30 mg/ml | 0.1 |

The batch is aerated for 60 minutes with 95% $0_2$/5% $CO_2$, the pH is checked to insure it is at 7.4 ± 0.1.

2. Add 0.32 M sucrose: 21.9 g of sucrose, q.s. to 200 ml.

3. A 0.1mM stock solution of serotonin creatinine $SO_4$ is made up in 0.01 N HCl. This is used to dilute the specific activity of the radiolabeled 5HT.

4. 5-[1,2-$^3$H(N)]-Hydroxytryptamine creatinine sulfate (serotonin) specific activity 20-30 Ci/mmol is used.

The final desired concentration of $^3$H-5HT in the assay is 50 nM. The dilution factor is 0.8. The KHBB is made up to contain 62.5 nM of [$^3$H]-5HT.

Add to 100 ml of KHBB.

| | |
|---|---|
| A) 56.1 µl of 0.1 mM 5HT | = 56.1nM |
| B) 0.64 nmol of $^3$H-5HT | = 6.4nM |
| | 62.5nM |

5. For most assays, a 1mM stock solution of the test compound is made up in a suitable solvent and serially diluted such that the final concentration in the assay ranges from 2 X $10^{-8}$ to 2 X $10^{-5}$M. Seven concentrations are used for each assay.

C. TISSUE PREPARATION

Male Wistar rats are decapitated and the brain rapidly removed. Either whole brain minus cerebella or hypothalmus is weighed and homogenized in 9 volumes of ice-cold 0.32 M sucrose using a Potter-Elvejhem homogenizer. The homogenate is centrifuged at 1000 g for 10 minutes at 0-4°C. The supernatant ($S_1$) is decanted and is used for uptake determination.

D. ASSAY

| | |
|---|---|
| 800 µl | KHBB + [$^3$H]-5HT |
| 20 µl | Vehicle or appropriate drug |
| 200 µl | Tissue suspension concentration |

Tubes are incubated at 37°C under a 95%$O_2$/5% $CO_2$ atmosphere for 5 minutes. For each assay, 3 tubes are incubated with 20 µl of vehicle at 0°C in an ice bath. After incubation all tubes are immediately centrifuged at 4000 g for 10 minutes. The supernatant fluid is aspirated and the pellets dissolved by adding 1 ml of solubilizer (Triton X-100 and 50% ethanol, 1:4 v/v). The tubes are vigorously vortexted, decanted into scintillation vials, and counted in 10 ml of Liquiscint scintillation counting cocktail. Active uptake is the difference between cpm at 37°C and 0°C. The per cent inhibition at each drug concentration is the mean of three determinations. $IC_{50}$ values are derived from log-probit analysis.

The results of this assay for representative compounds of this invention as well as reference compounds are presented in Table 2.

TABLE 2

| COMPOUND | 5-HT-$IC_{50}$(μM) |
|---|---|
| [4-[2-[3-[1,2-Benzisoxazol-3-yl]-8-azabicyclo[3.2.1]octan-8-yl]ethoxy]-3-methoxyphenyl] ethanone fumarate | 0.01 |
| [4-[4-[3-[1H-Indazol-3-yl]-8-azabicyclo[3.2.1]octan-8-yl]butoxy]-3-methoxyphenyl]ethanone fumarate hemihydrate | 0.07 |
| [4-[4-[3-[6-Fluoro-1H-indazol-3-yl]-8-azabicyclo[3.2.1]-octan-8-yl]butoxy]-3-methoxyphenyl] ethanone | 0.02 |
| [4-[4-[3-[1,2-Benzisothiazol-3-yl]-8-azabicyclo[3.2.1]octan-8-yl]butoxy]-3-methoxyphenyl] ethanone monohydrochloride | 0.027 |
| Chloripramine (reference) | 0.15 |
| Fluoxetine (reference) | 0.247 |

Antidepressive response is achieved when the compounds of the present invention are administered to a subject requiring such treatment as an effective oral, parenteral, or intravenous dose of from 1 to 100 mg/kg of body weight per day. It is to be understood, however, that for any particular subject, specific dosage regimens should be adjusted according to the individual need and the professional judgment of the person administering or supervising the administration of the aforesaid compound. It is to be further understood that the dosages set forth herein are exemplary only and they do not, to any extent, limit the scope or practice of the invention.

Effective quantities of the compounds of the present invention may be administered to a subject by any one of various methods, for example, orally as in capsules or tablets, parenterally in the form of sterile solutions or suspensions, and in some cases intravenously in the form of sterile solutions.

The compounds of the present invention, while effective themselves, may be formulated and administered in the form of their pharmaceutically acceptable acid addition salts for purposes of stability, convenience of crystallization, increased solubility and the like.

Preferred pharmaceutically acceptable addition salts include salts of inorganic acids such as hydrochloric, hydrobromic, sulfuric, nitric,phosphoric and perchloric acids; as well as organic acids such as tartaric, citric, acetic, succinic, maleic, fumaric, and oxalic acids.

The active compounds of the present invention may be administered orally, for example, with an inert diluent or with an edible carrier. They may be enclosed in gelatin capsules or compressed into tablets. For the purpose of oral therapeutic administration, the compounds may be incorporated with excipients and used in the form of tablets, troches, capsules, elixirs, suspensions, syrups, wafers, chewing gums and the like. These preparations should contain at least 0.5% of active compound, but may be varied depending upon the particular form and may conveniently be between 4% to about 75% of the weight of the unit. The amount of compound present in such composition is such that a suitable dosage will be obtained. Preferred compositions and preparations according to the present invention are prepared so that an oral dosage unit form contains between 1.0-300 mgs of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel™, corn starch and the like; a lubricant such as magnesium stearate or Sterotex®; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain,in addition to materials of the above type, a liquid carrier such as fatty oil. Other dosage unit forms may contain other various materials which modify the physical form of the doseage unit, for example, as coatings. Thus tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0.1% of the aforesaid compound, but may be varied between 0.5 and about 30% of the weight thereof. The amount of active compound in such compositions is such that a suitable dosage will be obtained. Preferred compositions and preparations according to the present invention are prepared so that a parenteral dosage unit contains between 0.5 to 100 mgs of active compound.

The solutions or suspensions may also include the following components; a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating

agents such as ethylenediaminetetraacetic acid; buffers such as acetates,citrates or phosphates and agents for the adjustment of tonicity such as sodium chloride or dextrose. The parenteral preparation can be enclosed in ampules, disposable syringes or multiple dose vials made of glass or plastic.

Following are typical examples of compounds of the invention that can be prepared by following the methods of preparation described earlier:

[4-[2-[3-[6-fluoro-1,2-benzisothiazol-3-yl]-8-azabicyclo[3.2.1]octan-8-yl]propoxy]-3-methoxyphenyl]ethanone;
[4-[2-[3-[6-fluoro-1,2-benzisoxazol-3-yl]-8-azabicyclo[3.2.1]octan-8-yl]propoxy]-2-methylphenyl]ethanone;
[4-[2-[3-[6-fluoro-1,2-benzisoxazol-3-yl]-8-azabicyclo[3.2.1]octan-8-yl]propoxy]-2-methoxyphenyl]ethanone;
[4-[2-[3-[6-fluoro-1,2-benzisoxazol-3-yl]-8-azabicyclo[3.2.1]octan-8-yl]propoxy]-3-methylaminophenyl]ethanone;
N-[4-[2-[3-[6-fluoro-1,2-benzisoxazol-3-yl]-8-azabicyclo[3.2.1]octan-8-yl]propoxy]-3-methoxyphenyl]acetamide;
[4-[2-[3-[6-chloro-1,2-benzisoxazol-3-yl]-8-azabicyclo[3.2.1]octan-8-yl]propoxy]-3-methoxyphenyl]ethanone;
[4-[2-[3-[6-fluoro-1,2-benzisoxazol-3-yl]-8-azabicyclo[3.2.1]octan-8-yl]propoxy]-3-methoxybenzonitrile;
[4-[2-[3-[6-fluoro-1,2-benzisoxazol-3-yl]-8-azabicyclo[3.2.1]octan-8-yl]propoxy]-3-bromophenyl]ethanone;
[4-[2-[3-[6-fluoro-1,2-benzisoxazol-3-yl]-8-azabicyclo[3.2.1]octan-8-yl]propoxy]-3-methylmercaptophenyl]ethanone;
[4-[2-[3-[6-fluoro-1,2-benzisothiazol-3-yl]-8-azabicyclo[3.2.1]octan-8-yl]butoxy]-3-methoxyphenyl]ethanone;
[4-[2-[3-[6-fluoro-1,2-benzisothiazol-3-yl]-8-azabicyclo[3.2.1]octan-8-yl]ethoxy]-3-methoxyphenyl]ethanone; and
[4-[2-[3-[6-fluoro-1,2-benzisoxazol-3-yl]-8-azabicyclo[3.2.1]octan-8-yl]propoxyl]phenyl]ethanone.

The following examples are for illustrative purposes only and are not to be construed as limiting the invention. All temperatures are given in degrees centrigrade (°C) unless indicated otherwise.

**Example 1**

a. **Diethyl-1-(2-fluorophenyl)-1-methoxymethane phosphonate**

Boron trifluoride etherate (71 g) was added dropwise to a solution of 2-fluorobenzaldehyde dimethyl acetal (81 g) and triethylphosphite (79 g) in 950 ml of dichloromethane at -25°C. The resulting solution was allowed to warm to room temperature and stirred for 20 hours. Water was added, and the mixture was stirred vigorously for 10 minutes. The organic layer was separated, washed with brine, dried over $MgSO_4$, filtered and concentrated to leave a liquid. Purification by HPLC on silica gel (elution with dichloromethane, followed by 1:1 ethyl acetate-dichioromethane) provided 96.9 g of diethyl-1-(2-fluorophenyl)-1-methoxymethane phosphonate, as a liquid.

b. **(2-Fluorophenyl)(8-methyl-8-azabicyclo[3.2.1]octan-3-yl)methanone**

Diethyl-1-(2-fluorophenyl)-1-methoxymethane phosphonate (96 g) was dissolved in tetrahydrofuran (THF) (1600 ml). The solution was cooled to -78°C and n-butyllithium (n-BuLi) (172 ml of a 2.5 M solution in hexanes) was added dropwise at a rate such that the temperature never rose above -65° C. The resulting solution was stirred for 1 hour. Tropinone (44 g) dissolved in THF(100 ml) was then added slowly dropwise to the reaction mixture. After complete addition, the mixture was allowed to warm slowly to room temperature and was stirred overnight. Water was added to the reaction mixture, the layers were separated, and the organic layer dried over $MgSO_4$, filtered and concentrated to yield an oil. The oil was dissolved in acetone (2L). Water (350 ml) and concentrated HCl (18 ml) were added and the mixture was refluxed for 3 hours. The acetone was removed *in vacuo,* and the aqueous phase was extracted with ethyl acetate, basified with $K_2CO_3$ and the product was extracted into dichloromethane. The combined organic layers were washed with brine, dried over $MgSO_4$, filtered and concentrated to leave 72 g of (2-fluorophenyl)(8-methyl-8-azabicyclo [3.2.1]octan-3-yl)methanone, as a solid.

c. **Z-(2-Fluorophenyl)(8-methyl-8-azabicyclo[3.2.1] octan-3-yl)methanone oxime hydrochloride**

A mixture of (2-fluorophenyl)(8-methyl-8-azabicyclo[3.2.1]octan-3-yl)methanone (29.5 g), hydroxylamine hydrochloride (16.5 g) and ammonium acetate (27.5 g) were heated in 80 ml of refluxing ethanol-water (3:1 mixture) for 19 hours. The mixture was cooled and the precipitated product was collected (25.2g).

d. **3-(1,2-Benzisoxazol-3-yl)-8-methyl-8-azabicyclo[3.2.1]octane hydrochloride monohydrate**

A solution of (2-fluorophenyl)(8-methyl-8-azabicyclo[3.2.1]octan-3-yl)methanone oxime (14 g) in 56 ml of 10% sodium hydroxide solution and 140 ml of ethanol was heated at reflux for 4 hours. The resulting solution was cooled,

diluted with water, and the product was extracted into dichloromethane. The combined organic layers were washed with brine, dried over magnesium sulfate, filtered, and concentrated to provide 13.1 g of an oil. The crude product was dissolved in methanol and acidified. The volume of solvent was reduced, ethyl acetate was added, and the product crystallized from solution. The product was collected by filtration, affording 5.5 g of 3-(1,2-benzisoxazol-3-yl)-8-methyl-8-azabicyclo[3.2.1]octane hydrochloride monohydrate, as crystals, m.p. 232-233°C.

| Analysis: | | | |
|---|---|---|---|
| Calculated for $C_{15}H_{21}ClN_2O_2$: | 60.70%C | 7.13%H | 9.44%N |
| Found: | 60.83%C | 6.78%H | 9.42%N |

**e. 3-(1,2-Benzisoxazol-3-yl)-8-azabicyclo-[3.2.1]octane hydrochloride**

Vinyl chloroformate (3.6 g) was added dropwise to a solution of 3-(1,2-benzisoxazol-3-yl)-8-methyl-8-azabicyclo [3.2.1]octane (6.0 g) in 125 ml of 1,2-dichloroethane at 0°C. The resulting suspension was heated at reflux for 3 hours, the solution was cooled, and the solvent was removed *in vacuo*. The residue was suspended in 125 ml of ethanol and acidified with HCl in isopropanol (to about a pH of 1), and the mixture was heated at reflux for 2 hours. The mixture was cooled, and 3.9 g of 3-(1,2-benzisoxazol-3-yl)-8-azabicyclo[3.2.1]octane hydrochloride crystallized, m.p. 264-268°C.

| Analysis: | | | |
|---|---|---|---|
| Calculated for $C_{14}H_{17}ClN_2O$: | 63.51%C | 6.47%H | 10.58%N |
| Found: | 63.67%C | 6.51%H | 10.42%N |

**f. [4-[4-[3-[1,2-Benzisoxazol-3-yl]-8-azabicyclo[3.2.1]octan-8-yl]butoxy]-3-methoxyphenyl]ethanone hydrochloride**

A mixture of 3-(1,2-benzisoxazol-3-yl)-8-azabicyclo[3.2.1]octane (3.7 g), 4-(4-bromobutoxy)-3-methoxyphenyl ethanone (5.3 g), and potassium carbonate (4.5 g) was heated in 65 ml of refluxing acetonitrile for 22 hours. The resulting mixture was allowed to cool to room temperature and then filtered. The filtrate was diluted with dichloromethane, and then washed sequentially with water and brine, dried over $K_2CO_3$, filtered, and concentrated to provide 7.1 g of crude product. Purification by HPLC on silica gel (elution with ethyl acetate) afforded 4.6 g of product as an oil. The oil was dissolved in hot ethanol and treated with a solution of HCl in isopropanol. Isopropyl ether was added to the solution, and the hydrochloride was allowed to crystallize. Filtration gave 4.48 g of[4-[4-[3-[1,2-benzisoxazol-3-yl]-8-azabicyclo [3.2.1]octan-8-yl]butoxy]-3-methoxyphenyl] ethanone hydrochloride, m.p. 216.5-218°C.

| Analysis: | | | |
|---|---|---|---|
| Calculated for $C_{27}H_{33}ClN_2O_4$: | 66.86%C | 6.86%H | 5.78%N |
| Found: | 66.78%C | 7.11%H | 5.53%N |

**Example 2**

**a. Diethyl-1-(2,4-difluorophenyl)-1-methoxymethane phosphonate**

Boron trifluoride etherate (86 g) was added dropwise to a solution of 2,4-difluorobenzaldehyde dimethyl acetal (114 g) and triethylphosphite (101 g) in 1.2l of dichloromethane at -25°. The resulting solution was allowed to warm to room temperature and stirred for 20 hours. Water was added, and the mixture was stirred vigorously for 10 minutes. The organic layer was separated, washed with brine, dried over $MgSO_4$, filtered and concentrated to leave a liquid. Purification by HPLC on silica gel (elution with dichloromethane, followed by 5% ethyl acetate-dichloromethane) provided 136 g of diethyl-1-(2,4-difluorophenyl)-1-methoxymethane phosphonate, as a liquid.

**b. (2,4-Difluorophenyl)(8-methyl-8-azabicyclo[3.2.1]octan-3-yl)methanone hydrochloride**

Diethyl-1-(2,4-difluorophenyl))-1-methoxymethane phosphonate (76 g) was dissolved in THF (1600 ml). The solution was cooled to -78°C and n-BuLi (103.4 ml of a 2.5M solution in hexanes) was added dropwise at a rate such that the temperature never rose above -65°C. The resulting solution was stirred for 1 hour. Tropinone (32.7 g) dissolved

in THF(100 ml) was then added slowly dropwise to the reaction mixture. After complete addition, the mixture was allowed to warm slowly to room temperature and was stirred overnight. A saturated NaCl solution (1.5 L) was added to the reaction mixture. The layers were separated and the organic layer was collected and dried over $MgSO_4$. The solvent was removed via rotary evaporation to yield an oil (73 g). This oil (38 g) was dissolved in acetone (2L). Water (350 ml) and concentrated HCl (182 ml) were added slowly and the mixture was refluxed for 3 hours. The acetone was removed via rotary evaporation. The aqueous residue which remained was extracted with ethyl acetate, basified with $K_2CO_3$, extracted with dichloromethane (DCM), and dried over $MgSO_4$. Evaporation of the solvent yielded 31.3 g of an oil which solidified. A portion of this solid (3 g) was dissolved in ethanol (75 ml). Ethanolic HCl was added until the solution was acidic. Ethyl ether (75 ml) was added and the product salt (2.65 g, m.p. 224-225°C) precipitated from solution.

| Analysis: | | | |
|---|---|---|---|
| Calculated for $C_{15}H_{18}ClF_2NO$: | 59.70%C | 6.01%H | 4.64%N |
| Found: | 59.52%C | 5.87%H | 4.55%N |

**c. Z-(2,4-Difluorophenyl)(8-methyl-8-azabicyclo[3.2.1] octan-3-yl)methanone oxime hydrochloride**

A mixture of (2,4-difluorophenyl)(8-methyl-8-azabicyclo[3.2.1]octan-3-yl)methanone (20 g), hydroxylamine hydrochloride (10.6 g) and ammonium acetate (18.7 g) was heated in 67.5 ml of refluxing ethanol-water (3.2 mixture) for 19 hours. The mixture was concentrated to approximately half of its original volume, and the precipitated solid (21.3 g) was collected.

**d. 3-[6-Fluoro-1,2-benzisoxazol-3-yl]-8-methyl-8-azabicyclo[3.2.1]octane hydrochloride**

A mixture of (E)-(2,4-difluorophenyl)(8-methyl-8-azabicyclo[3.2.1]octan-3-yl)methanone oxime (18 g), 10% NaOH (36.4 ml) and ethanol (150 ml) was refluxed for 4 hours. The reaction mixture was cooled and concentrated on the rotary evaporator. The mixture was diluted with $H_2O$ (500 ml) and extracted with ethyl ether (2x1L). The ether extract was dried with $MgSO_4$ and concentrated to yield an oil (13.6 g) which solidified upon standing several hours. A portion of this solid (3.0 g) was dissolved in ethanol and ethanolic HCl was added dropwise until the solution was acidic. 2.1 g of 3-[6-fluoro-1,2-benzisoxazol-3-yl]-8-methyl-8-azabicyclo[3.2.1] octane hydrochloride, m.p. 269-270°C, precipitated from solution.

| Analysis: | | | |
|---|---|---|---|
| Calculated for $C_{15}H_{17}FN_2O{\cdot}HCl$: | 60.71%C | 6.11%H | 9.44%N |
| Found: | 60.83%C | 6.17%H | 9.33%N |

**e. 3-(6-Fluoro-1,2-benzisoxazol-3-yl)-8-azabicyclo[3.2.1]octane hydrochloride**

Vinyl chloroformate (2.9 g) was added dropwise to a solution of 3-(6-fluoro-1,2-benzisoxazol-3-yl)-8-methyl-8-azabicyclo[3.2.1]octane (5.6 g) and potassium carbonate (3.6 g) in 125 ml of 1,2-dichioroethane at 0°C. The resulting suspension was heated at reflux for 3 hours, the solution was cooled, and the solvent was removed *in vacuo*. The residue was suspended in 125 mL of ethanol and acidified with HCl in ethanol (to about a pH of 1), and the mixture was heated at reflux for 2 hours. The mixture was cooled, and 3.6 g of 3-(6-fluoro-1,2-benzisoxazol-3-yl)-8-azabicyclo [3.2.1]octane hydrochloride, m.p. 248-250°C, crystallized out of solution.

| Analysis: | | | |
|---|---|---|---|
| Calculated for $C_{14}H_{16}ClFN_2O$: | 59.46%C | 5.71%H | 9.91%N |
| Found: | 59.59%C | 5.73%H | 9.83%N |

**f. [4-[3-[3-[6-Fluoro-1,2-benzisoxazol-3-yl]-8-azabicyclo[3.2.1]octan-8-yl]propoxy]-3-methoxyphenyl]ethanone hydrochloride**

A mixture of 3-[6-fluoro-1,2-benzisoxazol-3-yl]-8-azabicyclo[3.2.1]octane (3.3 g), $K_2CO_3$ (2.21 g), 4-(3-chloropropoxy)-3-methoxyphenyl ethanone (3.9 g) and acetonitrile (150 ml) was stirred and refluxed for 18 hours. The mixture was filtered and the filtrate was evaporated under reduced pressure. The residue was purified using Prep 500 chromatography (5% methanol/1% triethylamine/94% DCM) to yield 3.4 g of an oil. The oil was dissolved in ethanol (40 ml) and ethanolic HCl was added until the solution was acidic. Ethyl ether (approximately 100 ml) was added and 2.4 g of [4-[3-[3-[6-fluoro-1,2-benzisoxazol-3-yl]-8-azabicyclo[3.2.1]octan-8-yl]propoxy]-3-methoxyphenyl]ethanone hydrochioride precipitated from solution as a solid, m.p. 219-220°C.

| Analysis: | | | |
|---|---|---|---|
| Calculated for $C_{26}H_{30}ClFN_2O_4$: | 63.86%C | 6.18%H | 5.73%N |
| Found: | 63.69%C | 6.09%H | 5.60%N |

**Example 3**

**[4-[3-[3-[1,2-Benzisoxazol-3-yl]-8-azabicyclo[3.2.1]octan-8-yl]propoxy]-3-methoxyphenyl] ethanone fumarate**

A mixture of 3-(1,2-benzisoxazol-3-yl)-8-azabicyclo[3.2.1]octane (3.2. g), 4-(3-chloropropoxy)-3-methoxyphenylphenyl ethanone (4.0 g), potassium carbonate (3.9 g) and a catalytic amount of potassium iodide was heated in 60 ml of refluxing acetonitrile for 19 hours. The resulting mixture was allowed to cool to room temperature, diluted with water and extracted into dichloromethane. The organic layer was washed with brine, dried with $MgSO_4$, filtered, and concentrated to give 7.1 g of an oil, which was dissolved in diethyl ether and acidified with HCl in isopropanol. The solid was collected by filtration, suspended in water, and basified with 10% NaOH solution. The product was extracted into dichloromethane, and then washed sequentially with water and brine, dried over $K_2CO_3$, filtered, and concentrated to provide 4.0 g of crude product. Purification by HPLC on silica gel (elution with ethyl acetate) afforded 2.7 g of product as an oil. The oil (2.5 g) was dissolved in hot ethanol, and it was treated with an equivalent amount of fumaric acid. Isopropyl ether was added to the solution, and the fumarate was allowed to crystallize. Filtration gave 2.85 g of [4-[3-[3-[1,2-benzisoxazol-3-yl]-8-azabicyclo[3.2.1]octan-8-yl]propoxy]-3-methoxyphenyl] ethanone fumarate, m.p. 176-178°C.

| Analysis: | | | |
|---|---|---|---|
| Calculated for $C_{30}H_{34}N_2O_8$: | 65.44%C | 6.22%H | 5.09%N |
| Found: | 65.11%C | 6.25%H | 5.06%N |

**Example 4**

**[4-[2-[3-[6-Fluoro-1,2-benzisoxazol-3-yl]-8-azabicyclo[3.2.1] octan-8-yl]ethoxy]-3-methoxyphenyl] ethanone fumarate**

A mixture of 3-(6-fluoro-1,2-benzisoxazol-3-yl)-8-azabicyclo[3.2.1]octane (3.0 g), 4-(2-chloroethoxy)-3-methoxyphenyl ethanone (3.6 g), and potassium carbonate (2.2 g), was heated in 200 ml of refluxing acetonitrile for 22 hours. The resulting mixture was allowed to cool to room temperature and filtered. The filtrate was concentrated and purified by HPLC on silica gel (elution with triethylamine- methanol- ethyl acetate) to afford 2.6 g of product as an oil. The oil was dissolved in methanol, and fumaric acid (0.76 g) was added. The product crystallized from solution upon addition of ethyl ether to afford 2.0 g of [4-[2-[3-[6-fluoro-1,2-benzisoxazol-3-yl]-8-azabicyclo[3.2.1]octan-8-yl]ethoxy]-3-methoxyphenyl]ethanone fumarate, as a powder, m.p. 171-172°C.

| Analysis: | | | |
|---|---|---|---|
| Calculated for $C_{29}H_{31}FN_2O_8$: | 62.81%C | 5.63%H | 5.05%N |
| Found: | 62.69%C | 5.61%H | 5.02%N |

**Example 5**

**[4-[2-[3-[1,2-Benzisoxazol-3-yl]-8-azabicyclo[3.2.1]octan-8-yl] ethoxy]-3-methoxyphenyl] ethanone fumarate**

A mixture of 3-(1,2-benzisoxazol-3-yl)-8-azabicyclo[3.2.1]octane (3.4 g), 4-(2-chloroethoxy)-3-methoxyphenyl ethanone (4.1 g), potassium carbonate (4.1 g) and a catalytic amount of potassium iodide was heated in 60 ml of refluxing acetonitrile for 19 hours. The resulting mixture was allowed to cool to room temperature and then was filtered. The filtrate was concentrated, and the residue was dissolved in diethyl ether and acidified with HCl in isopropanol. The

solid was collected by filtration, suspended in water, and basified with 10% NaOH solution. The product was extracted into dichloromethane, and then washed sequentially with water and brine, dried over $K_2CO_3$, filtered, and concentrated to provide 4.7 g of crude product. Purification by HPLC on silica gel (elution with ethyl acetate) afforded 2.8 g of product as an oil. The oil (2.55 g) was dissolved in hot ethanol, and the solution was treated with an equivalent amount of fumaric acid. Isopropyl ether was added to the solution, and the fumarate was allowed to crystallize. Filtration gave 2.95 g of [4-[2-[3-[1,2-benzisoxazol-3-yl]-8-azabicyclo[3.2.1]octan-8-yl]ethoxy]-3-methoxyphenyl] ethanone fumarate, m.p. 182-183.5°C.

| Analysis: | | | |
|---|---|---|---|
| Calculated for $C_{29}H_{32}N_2O_8$: | 64.91%C | 6.01%H | 5.22%N |
| Found: | 64.92%C | 6.02%H | 5.20%N |

**Example 6**

**[4-[4-[3-[6-Fluoro-1,2-benzisoxazol-3-yl]-8-azabicyclo[3.2.1]octan-8-yl]butoxy]-3-methoxyphenyl] ethanone fumarate**

A mixture of 3-(6-fluoro-1,2-benzisoxazol-3-yl)-8-azabicyclo[3.2.1]octane(3.5 g), 4-(4-bromobutoxy)-3-methoxyphenyl ethanone (5.1 g), and potassium carbonate (2.4 g) was heated in 200 ml of refluxing acetonitrile for 22 hours. The resulting mixture was allowed to cool to room temperature and filtered. The filtrate was concentrated and purified by HPLC on silica gel (elution with triethylamine- methanol- ethyl acetate) to afford 5.3 g of product as an oil. The oil was dissolved in methanol and fumaric acid (1.4 g) was added. The product crystallized upon addition of ethyl ether affording 4.2 g of [4-[4-[3-[6-fluoro-1,2-benzisoxazol-3-yl]-8-azabicyclo[3.2.1]octan-8-yl]butoxy]-3-methoxyphenyl] ethanone fumarate, as a powder, m.p. 139-141°C.

| Analysis: | | | |
|---|---|---|---|
| Calculated for $C_{31}H_{35}FN_2O_8$: | 63.90%C | 6.07%H | 4.81%N |
| Found: | 63.68%C | 5.95%H | 4.69%N |

**Example 7**

**a. 3-(1H-Indazol-3-yl)-8-methyl-8-azabicyclo[3.2.1]octane**

A mixture of (2-fluorophenyl) (8-methyl-8-azabicyclo-[3.2.1]octan-3-yl) methanone (24.6 g), hydrazine hydrate (14.4 ml), and ethanol (250 ml) was heated to reflux for four hours. The reaction was cooled to room temperature and concentrated to an oil. This residue was dissolved in dimethylformamide (DMF) (250 ml). Potassium carbonate (28 g) was added to the mixture which was subsequently heated at reflux for 2 days. The reaction mixture was cooled and filtered and the DMF was removed *in vacuo.* The residue was dissolved in ethanol and 5.2 g of 3-(1H-indazol-3-yl)-8-methyl-8-azabicyclo[3.2.1]octane precipitated from solution, as a powder, m.p. 191-192°C.

| Analysis: | | | |
|---|---|---|---|
| Calculate for $C_{15}H_{19}N_3$: | 74.64%C | 7.95%H | 17.41%N |
| Found: | 74.53%C | 8.02%H | 17.21%N |

**b. 3-(1H-Indazol-3-yl)-8-azabicyclo[3.2.1]octane**

Cyanogen bromide (18.2 g) was added in one portion to a suspension of (1H-indazol-3-yl)-8-methyl-8-azabicyclo [3.2.1]octane (20.7 g) and potassium carbonate (23.7 g) in 340 ml of DMF at room temperature. The mixture was stirred for 2 hours, and then was diluted with water, and the product was extracted into ethyl acetate. The combined organic layers were washed with water and brine, dried over $MgSO_4$, filtered and concentrated to leave 22.2 g of an oil. Trituration with ethyl acetate-hexanes provided 11.9 g of a solid.

A portion of the above cyanamide (6.8 g) in 130 ml of THF at 0°C was treated dropwise with lithium aluminum hydride (1 M in, THF, 57 ml). The resulting mixture was heated at reflux for 1 hour and then cooled to 0°C and quenched with water. The mixture was diluted with 7 ml of 20% NaOH solution and 7 ml of water. Sodium sulfate was added, the mixture was filtered and the filtrate concentrated to leave 6.2 g of a foam which was alkylated without further purification.

**c. [4-[3-[3-[1H-Indazol-3-yl]-8-azabicyclo[3.2.1]octan-8-yl]propoxy]-3-methoxyphenyl] ethanone**

A mixture of 3-(1H-indazol-3-yl)-8-azabicyclo[3.2.1]octane (5.46 g), 4-(3-chloropropoxy)-3-methoxyphenyl ethanone (6.4 g), potassium carbonate (6.6 g) and a catalytic amount of potassium iodide was heated in 100 ml of refluxing acetonitrile for 17 hours. The resulting mixture was allowed to cool to room temperature and filtered. The filtrate was concentrated, and the residue was dissolved in 6N HCl solution and extracted with ethyl acetate. The aqueous layer was basified with 10% NaOH solution, and the product was extracted into dichloromethane. The combined organic layers were washed with brine, dried over $K_2CO_3$, filtered, and concentrated to provide 7.0 g of a foam. Purification by HPLC on silica gel (elution initially with ethyl acetate, and then 10% methanol-90% ethyl acetate) afforded 4.2 g of product as a foam. The foam crystallized upon the addition of ethyl acetate, and the solid was then recrystallised from ethyl acetate-hexanes, affording 2.8 g of [4-[3-[3-[1H-indazol-3-yl]-8-azabicyclo[3.2.1]octan-8-yl]propoxyl]-3-methoxyphenyl] ethanone, as a powder, m.p. 173-175°C.

| Analysis: | | | |
|---|---|---|---|
| Calculated for $C_{26}H_{31}N_3O_3$: | 72.03%C | 7.21%H | 9.69%N |
| Found: | 71.69%C | 7.14%H | 9.64%N |

**Example 8**

**[4-[2-[3-[1H-Indazol-3-yl]-8-azabicyclo[3.2.1]octan-8-yl]-ethoxy]-3-methoxyphenyl] ethanone**

A mixture of 3-(1H-indazol-3-yl)-8-azabicyclo[3.2.1]octane (4.1 g), 4-(2-chloroethoxy)-3-methoxyphenyl ethanone (5.9 g) and potassium carbonate (2.7 g) was heated in 125 ml of refluxing acetonitrile for 22 hours. The resulting mixture was allowed to cool to room temperature and then filtered. The filtrate was concentrated and purified by HPLC on silica gel (elution with triethylamine- methanol- ethyl acetate) to afford 2.3 g of product as an oil. The oil was dissolved in ethyl acetate and the product crystallized affording 1.6 g of [4-[2-[3-[1H-indazol-3-yl]-8-azabicyclo[3.2.1]octan-8-yl] ethoxy]-3-methoxyphenyl] ethanone, as a powder, m.p. 154-155°C.

| Analysis: | | | |
|---|---|---|---|
| Calculated for $C_{25}H_{29}N_3O_3$: | 71.56%C | 6.98%H | 10.02%N |
| Found: | 71.42%C | 6.95%H | 9.98%N |

**Example 9**

**[4-[4-[3-[1H-Indazol-3-yl]-8-azabicyclo[3.2.1]octan -8-yl]butoxy]-3-methoxyphenyl] ethanone fumarate hemihydrate**

A mixture of 3-(1H-indazol-3-yl)-8-azabicyclo[3.2.1]octane (3.1 g), 4-(4-bromobutoxy)-3-methoxyphenyl ethanone (4.52 g) and potassium carbonate (3.7 g) was heated in 56 ml of refluxing acetonitrile for 22 hours. The resulting mixture was allowed to cool to room temperature and then filtered. The filtrate was diluted with dichloromethane, and then washed sequentially with water and brine, dried over $K_2CO_3$, filtered, and concentrated to provide 6.0 g of crude product. Purification by HPLC on silica gel (elution with triethylamine- methanol- ethyl acetate) afforded 3.4 g of product as an oil. The oil was dissolved in ethanol, and the solution was treated with an equivalent amount of fumaric acid. The solvent was removed *in vacuo*, and the resulting foam was crystallized from water, affording 3.3 g of [4-[4-[3-[1H-indazol-3-yl]-8-azabicyclo[3.2.1]octan-8-yl]butoxy]-3-methoxyphenyl] ethanone fumarate hemihydrate, as a powder, m.p. 170-173°C.

| Analysis: | | | |
|---|---|---|---|
| Calculated for $C_{31}H_{37}N_3O_7 \cdot 0.5H_2O$: | 65.02%C | 6.69%H | 7.34%N |
| Found: | 65.08%C | 6.74%H | 7.22%N |

**Example 10**

**a. 3-(6-Fluoro-1H-Indazol-3-yl)-8-methyl-8-azabicyclo[3.2.1]octane**

A mixture of (2,4-difluorophenyl)(8-methyl-8-azabicyclo-[3.2.1]octane-3-yl)methanone (31.2 g), hydrazine hydrate (14.2 ml), and ethanol (300 ml) was heated to reflux for 5 hours. The reaction was cooled to room temperature, concentrated, diluted with water and extracted into a 1:4 mixture of isopropanol-chloroform. Concentration gave 27 g of an oil. Part of this residue (17.7 g) was dissolved in DMF (180 ml). Potassium carbonate (16.8 g) was added to the mixture which was subsequently heated at reflux for 20 hours. The reaction mixture was cooled and filtered and the DMF was removed *in vacuo*. The residue was diluted with water and the product was extracted into isopropanol-chloroform (1:4). The combined organic layers were dried over magnesium sulfate, filtered and concentrated to give 18 g of crude product. Purification by HPLC (elution with ethyl acetate-methanol-triethylamine) gave 7.3 g of product.

**b. 3-(6-Fluoro-1H-indazol-3-yl)-8-azabicyclo[3.2.1]octane**

Cyanogen bromide (11.4 g) was added in one portion to a suspension of (6-fluoro-1H-indazol-3-yl)-8-methyl-8-azabicyclo[3.2.1]octane (23.4 g) and potassium carbonate (14.4 g) in 200 ml of DMF at room temperature. The mixture was stirred for 18 hours, filtered and concentrated. Water was added to the residue and the product was extracted into isopropanol-chloroform (1:4). The combined organic layers were dried over $MgSO_4$, filtered and concentrated. The residue was purified by HPLC (elution with ethyl acetate-methanol-triethylamine), providing 4.5 g of the cyanamide intermediate.

The cyanamide (14.3 g) in 125 ml of THF at 0°C, was treated dropwise with lithium aluminum hydride (1 M in THF, 105 ml). The resulting mixture was heated at reflux for 1 hour, and then cooled to 0°C and quenched with water. The mixture was diluted with 12 ml of 20% NaOH solution and 12 ml of water. The mixture was filtered through celite and concentrated. Water was added and the product was extracted into isopropanol-diethyl ether (1:4). The combined organics were dried over $MgSO_4$, filtered and concentrated to give 10.6 g of product which was alkylated without purification.

**c. [4-[2-[3-[6-Fluoro-1H-indazol-3-yl]-8-azabicyclo[3.2.1]octan-8-yl]ethoxy]-3-methoxyphenyl] ethanone**

A mixture of 3-(6-fluoro-1H-indazol-3-yl)-8-azabicyclo[3.2.1]octane (3.8 g), 4-(2-chloroethoxy)-3-methoxyphenyl ethanone (4.8 g) and potassium carbonate (4.8 g) was heated in 125 ml of refluxing acetonitrile for 22 hours. The resulting mixture was allowed to cool to room temperature and then filtered. The filtrate was concentrated and purified by HPLC on silica gel (elution with triethylamine- methanol- ethyl acetate) to afford 3.8 g of product as an oil. The oil was dissolved in isopropyl alcohol (IPA) and the product crystallized affording 2.1 g of [4-[2-[3-[6-fluoro-1H-indazol-3-yl]-8-azabicyclo[3.2.1]octan-8-yl]ethoxy]-3-methoxyphenyl] ethanone, as a powder, m.p. 151-152°C.

| Analysis: | | | |
|---|---|---|---|
| Calculated for $C_{25}H_{28}FN_3O_3$: | 68.63%C | 6.45%H | 9.60%N |
| Found: | 68.48%C | 6.45%H | 9.51%N |

**Example 11**

**[4-[4-[3-[6-Fluoro-1H-indazol-3-yl]-8-azabicyclo[3.2.1]octan-8-yl]butoxy]-3-methoxyphenyl] ethanone**

A mixture of 3-(6-fluoro-1H-indazol-3-yl)-8-azabicyclo[3.2.1]octane (6.0 g), 4-(4-bromobutoxy)-3-methoxyphenyl ethanone (7.5 g) and potassium carbonate (6.7 g) was heated in 150 ml of refluxing acetonitrile for 22 hours. The resulting mixture was allowed to cool to room temperature and then filtered. The filtrate was concentrated and purified by HPLC on silica gel (elution with triethylamine- methanol- ethyl acetate) to afford 4.6 g of product as an oil. The oil was dissolved in isopropyl alcohol, and the product crystallized affording 2.2 g of [4-[4-[3-[6-fluoro-1H-indazol-3-yl]-8-azabicyclo[3.2.1]octan-8-yl]butoxy]-3-methoxyphenyl] ethanone, as a powder, m.p. 148-149°C.

| Analysis: | | | |
|---|---|---|---|
| Calculated for $C_{27}H_{32}FN_3O_3$: | 69.66%C | 6.93%H | 9.03%N |
| Found: | 69.52%C | 7.09%H | 8.99%N |

**Example 12**

**[4-[3-[3-[6-Fluoro-1H-indazol-3-yl]-8-azabicyclo[3.2.1]octan-8-yl]propoxy]-3-methoxyphenyl] ethanone**

A mixture of 3-(6-fluoro-1H-indazol-3-yl)-8-azabicyclo[3.2.1]octane (4.3 g), 4-(3-chloropropoxy)-3-methoxyphenyl ethanone (4.7 g) and potassium carbonate (2.8 g) was heated in 150 ml of refluxing acetonitrile for 18 hours. The resulting mixture was allowed to cool to room temperature and then filtered. The filtrate was concentrated, and the residue was dissolved in $H_2O$ and extracted with 4:1 $CHCl_3$/IPA. The combined organic layers were dried over $Mg_2SO_4$, filtered and concentrated. Purification by HPLC on silica gel (elution initially with ethyl acetate, and then with 10% methanol-89% ethyl acetate-1% TEA) afforded 3.1 g of product as a foam. The foam was dissolved in methanol (50 ml) and the product crystallized affording 2.8 g of [4-[3-[3-[6-fluoro-1H-indazol-3-yl]-8-azabicyclo[3.2.1]octan-8-yl]propoxy]-3-methoxyphenyl] ethanone, as a solid, m.p. 157-158°C.

| Analysis: | | | |
|---|---|---|---|
| Calculated for $C_{26}H_{30}FN_3O_3$: | 69.14%C | 6.71%H | 9.31%N |
| Found: | 68.97%C | 6.99%H | 9.31%N |

**Example 13**

a. **3-[1,2-Benzisothiazol-3-yl]-8-methyl-8-azabicyclo[3.2.1]octane hydrochloride**

A mixture of (2-fluorophenyl)(8-methyl-8-azabicyclo-[3.2.1]octan-3-yl)methanone (20 g) and sulphur (3.2 g) in a saturated solution of $NH_3$ in 2-methoxyethanol (240 ml) was stirred in an autoclave at 160°C for 2 hours, and then cooled. The reaction mixture was poured into $H_2O$ (250 ml), extracted with DCM, and the organic phase was concentrated to an oil. This residue was purified by HPLC on silica gel (ethyl acetate/methanol/triethylamine) to yield an oil (6.3 g) which solidified upon standing. The solid (2.0 g) was dissolved in methanol. Ethereal HCl was added until the solution was acidic. Upon addition of ethyl ether, the product salt (1.7 g) precipitated from solution. Recrystallization twice from methanol yielded 0.5 g of 3-[1,2-benzisothiazol-3-yl]-8-methyl-8-azabicyclo[3.2.1]octane hydrochloride, m. p. 271-273°C.

| Analysis: | | | |
|---|---|---|---|
| Calculated for $C_{15}H_{19}ClN_2S$: | 61.09%C | 6.51%H | 9.50%N |
| Found: | 60.87%C | 6.49%H | 9.38%N |

b. **3-(1,2-Benzisothiazol-3-yl)-8-azabicyclo[3.2.1]octane**

Vinyl chloroformate (4.4 g) was added dropwise to a solution of 3-(1,2-benzisothiazol-3-yl)-8-methyl-8-azabicyclo [3.2.1]octane (8.9 g) and potassium carbonate (4.76 g) in 250 ml of 1,2-dichloroethane. The resulting suspension was heated at reflux for 3 hours, and then the solution was cooled, and the solvent was removed *in vacuo*. The residue was suspended in isopropyl alcohol and acidified with HCl in isopropanol (to about a pH of 1), and the mixture was heated at reflux for 1 hour. The mixture was cooled, made basic, and then the product was extracted into dichloromethane. The combined organic layers were dried over $MgSO_4$, filtered and concentrated to provide 8.5 g of 3-(1,2-benzisothiazol-3-yl)-8-azabicyclo[3.2.1]octane which was used subsequently without purification.

c. **[4-[2-[3-[1,2-Benzisothiazol-3-yl]-8-azabicyclo[3.2.1]octan-8-yl]ethoxy]-3-methoxyphenyl] ethanone fumarate**

A mixture of 3-(1,2-benzisothiazol-3-yl)-8-azabicyclo[3.2.1]octane (4.8 g), 4-(2-chloroethoxy)-3-methoxyphenyl ethanone (5.8 g) and potassium carbonate (3.5 g) was heated in 250 ml of refluxing acetonitrile for 22 hours. The resulting mixture was allowed to cool to room temperature and then filtered. The filtrate was concentrated and purified by HPLC on silica gel (elution with triethylamine- methanol-ethyl acetate) to afford 2.8 g of product as an oil. The oil was dissolved in ethanol and fumaric acid (0.82 g dissolved in ethanol) and was added to the free base in solution. The product (1.2 g) precipitated from solution upon addition of ethyl ether. The mother liquor was stripped of solvent, basified with NaOH and extracted with DCM. This crude free base was further purified by HPLC on silica gel to yield 1.2 g of an oil. The fumarate was prepared as before and was recrystallized from methanol to yield 0.8 g of the salt. The product samples were combined to yield 1.9 g of [4-[2-[3-[1,2-benzisothiazol-3-yl]-8-azabicyclo[3.2.1]octan-8-yl]

ethoxy]-3-methoxyphenyl] ethanone fumarate, as a solid, m.p 157-158°C.

| Analysis: | | | |
|---|---|---|---|
| Calculated for $C_{29}H_{32}N_2O_7S$: | 63.03%C | 5.84%H | 5.07%N |
| Found: | 62.95%C | 5.78%H | 5.00%N |

**Example 14**

**[4-[3-[3-[1,2-Benzisothiazol-3-yl]-8-azabicyclo[3.2.1]octan-8-yl]propoxy]-3-methoxyphenyl] ethanone fumarate**

A mixture of 3-(1,2-benzisothiazol-3-yl)-8-azabicyclo[3.2.1]octane (2.8 g), 4-(3-chloropropoxy)-3-methoxyphenyl ethanone (3.1 g), and potassium carbonate (1.8 g) was heated in 150 ml of refluxing acetonitrile for 22 hours. The resulting mixture was allowed to cool to room temperature and then filtered. The filtrate was concentrated and purified by HPLC on silica gel (elution with triethylamine- methanol- ethyl acetate) to afford 3.8 g of product as an oil. The oil was dissolved in methanol and fumaric acid (1.1 g dissolved in methanol) and was added to the free base in solution. The product (4.0 g) precipitated from solution upon addition of ethyl ether. This product was then recrystallized from methanol to yield 2.8 g of [4-[3-[3-[1,2-benzisothiazol-3-yl]-8-azabicyclo[3.2.1]octan-8-yl]propoxy]-3-methoxyphenyl] ethanone fumarate, m.p. 159-160°C.

| Analysis: | | | |
|---|---|---|---|
| Calculated for $C_{30}H_{34}N_2O_7S$: | 63.59%C | 6.05%H | 4.94%N |
| Found: | 63.83%C | 6.00%H | 5.00%N |

**Example 15**

**[4-[4-[3-[1,2-Benzisothiazol-3-yl]-8-azabicyclo[3.2.1]octan-8-yl]butoxyl-3-methoxyphenyl] ethanone hydrochloride**

A mixture of 3-(1,2-benzisothiazol-3-yl)-8-azabicyclo[3.2.1]octane (3.6 g), 4-(4-bromobutoxy)-3-methoxyphenyl ethanone (5.3 g) and potassium carbonate (2.5 g) was heated in 150 ml of refluxing acetonitrile for 22 hours. The resulting mixture was allowed to cool to room temperature and then filtered. The filtrate was concentrated and purified by HPLC on silica gel (elution with triethylamine- methanol- ethyl acetate) to afford 5.4 g of product as an oil. The oil was dissolved in methanol and ethereal HCl was added dropwise until the pH was acidic. The product crystallized from solution upon addition of additional ethyl ether affording 3.2 g of a powder. This product was then recrystallized from methanol to yield 2.1 g of [4-[4-[3-[1,2-benzisothiazol-3-yl]-8-azabicyclo[3.2.1]octan-8-yl]butoxy]-3-methoxyphenyl] ethanone hydrochloride, m.p. 205-206°C.

| Analysis: | | | |
|---|---|---|---|
| Calculated for $C_{27}H_{33}ClN_2O_3S$: | 64.70%C | 6.65%H | 5.59%N |
| Found: | 64.45%C | 6.63%H | 5.49%N |

**Claims**

**Claims for the following Contracting States : AT, BE, CH, LI, DE, DK, FR, GB, IT, LU, NL, PT, SE**

**1.** A compound of the formula

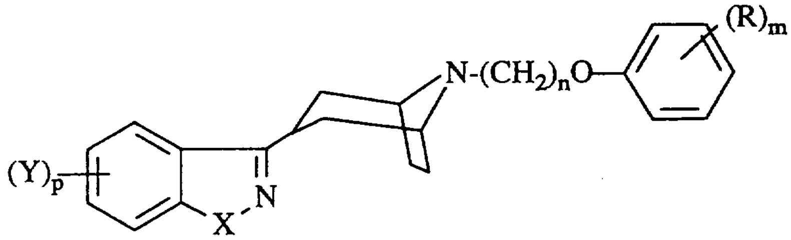

where

X is -O-, -S- or -NH-;
Y is hydrogen, halogen or loweralkoxy;
p is 1 or 2;
n is 2, 3 or 4;
R is hydrogen, loweralkyl, loweralkoxy, hydroxy, halogen, amino, loweralkylamino, nitro, loweralkylthio, trifluoromethoxy, cyano, trifluoromethyl,

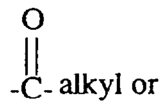 or 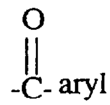

where aryl is

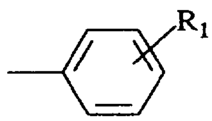 ;

$R_1$ is hydrogen, loweralkyl, loweralkoxy, halogen, hydroxy, carboxyl, loweralkylamino, nitro, loweralkylthio, cyano or trifluoromethyl;
m is 1 or 2; or a compound of the above formula where R is 3-methoxy and 1-acetamido, n is 3, is -O- and Y is 6-fluoro; or a pharmaceutically acceptable acid addition salt thereof or where applicable the geometric and optical isomers and racemic mixtures thereof.

**2.** A compound as defined in claim 1, wherein R is loweralkoxy or

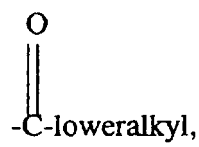,

Y is hydrogen or halogen, p is 1 and m is 2.

**3.** A compound as defined in claim 1, wherein X is O or S, n is 3, Y is hydrogen or fluoro.

**4.** A compound as defined in claim 1, wherein X is O and R is $OCH_3$ or

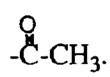.

**5.** The compound as defined in claim 1, which is [4-[2-[3-[1,2-benzisoxazol-3-yl]-8-azabicyclo[3.2.1]octan-8-yl]-ethoxy]-3-methoxyphenyl]ethanone or a pharmaceutically acceptable acid addition salt thereof.

**6.** The compound as defined in claim 1, which is [4-[4-[3-[1H-indazol-3-yl]-8-azabicyclo-[3.2.1]octan-8-yl] butoxy]-3-methoxyphenyl]-ethanone or a pharmaceutically acceptable acid addition salt thereof.

**7.** The compound as defined in claim 1, which is [4-[4-[3-[6-fluoro-1H-indazol-3-yl]-8-azabicyclo[3.2.1]octan-8-yl] butoxy]-3-methoxyphenyl]ethanone or a pharmaceutically acceptable acid addition salt thereof.

**8.** A pharmaceutical composition which comprises a compound as defined in claim 1 as the active ingredient and a

suitable carrier therefor.

**9.** Use of a compound as defined in claim 1 for the preparation of a medicament having antipsychotic and/or antidepressant activity.

**10.** A process for the preparation of a compound as defined in claim 1, which comprises reacting a compound of the formula, XI, XVI or XVIII

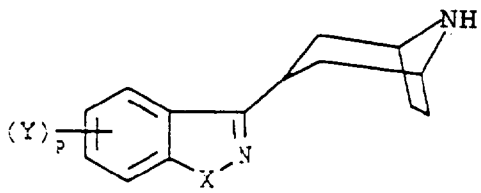

XI, XVI, XVIII

where X, Y and p are as defined with a compound of the formula XII

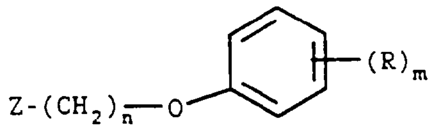

XII

where Z is chlorine or bromine and n is 2,3 or 4, and R and m are as defined, to afford a compound of the formula I, wherein X, Y, p, n, m and R are as defined.

**11.** A compound of the formula II

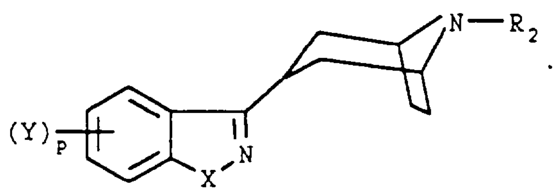

II

wherein $R_2$ is hydrogen or loweralkyl and X, Y and p are as defined in claim 1.

**Claims for the following Contracting States : ES, GR**

**1.** A process for the preparation of compound of the formula I

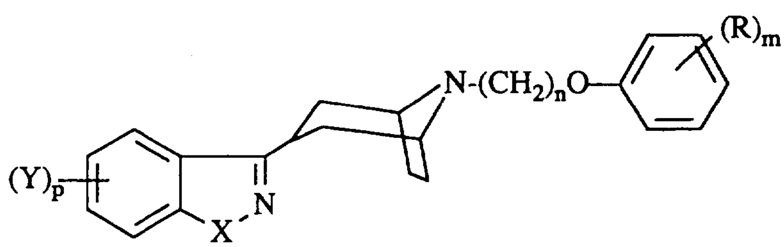

(I)

where

X is -O-, -S- or-NH-;
Y is hydrogen, halogen or lower alkoxy;
p is 1 or 2;
n is 2, 3 or 4;
R is hydrogen, loweralkyl, loweralkoxy, hydroxy, halogen, amino, loweralkylamino, nitro, loweralkylthio, trifluoromethoxy, cyano, trifluoromethyl,

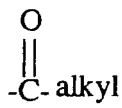 or 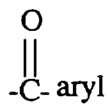

where aryl is

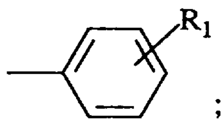 ;

$R_1$ is hydrogen, loweralkyl, loweralkoxy, halogen, hydroxy, carboxyl, loweralkylamino, nitro, loweralkylthio, cyano or trifluoromethyl;
m is 1 or 2; or a compound of the above formula where R is 3-methoxy and 1-acetamido, n is 3, X is O and Y is 6-fluoro; or a pharmaceutically acceptable acid addition salt thereof or where applicable the geometric and optical isomers and racemic mixtures thereof, which comprises
reacting a compound of the formula XI, XVI or XVIII

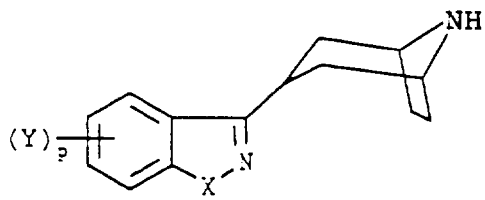 XI, XVI, XVIII

wherein X, Y and p are as defined with a compound of the formula XII

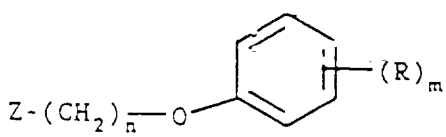 XII

where Z is chlorine or bromine and n is 2, 3 or 4, and R and m are as defined, to afford a compound of the formula I, wherein X, Y, p, n, m and R are as defined.

2. A process as defined in claim 1, wherein R is loweralkoxy or

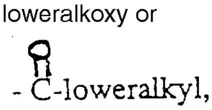,

loweralkyl, Y is hydrogen or halogen, p is 1 and m is 2.

3. A process as defined in claim 1, wherein X is O or S, n is 3 and Y is hydrogen or fluoro.

4. A process as defined in claim 1, wherein X is O and R is $OCH_3$ or

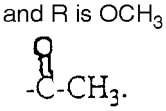.

5. The process as defined in claim 1, wherein [4-[2-[3-[1,2-benzisoxazol-3-yl]-8-azabicydo[3.2.1]octan-8-yl]-ethoxy]-3-methoxyphenyl]ethanone or a pharmaceutically acceptable acid addition salt thereof is prepared.

6. The process as defined in claim 1, wherein [4-[4-[3-[1H-indazol-3-yl]-8-azabicyclo-[3.2.1]octan-8-yl] butoxy]-3-methoxyphenyl]-ethanone or a pharmaceutically acceptable acid addition salt thereof is prepared.

7. The process as defined in claim 1, wherein [4-[4-[3-[6-fluoro-1H-indazol-3-yl]-8-azabicyclo[3.2.1]octan-8-yl] butoxy]-3-methoxyphenyl]ethanone or a pharmaceutically acceptable acid addition salt thereof is prepared.

8. Use of a compound as defined in claim 1 for the preparation of a medicament having antipsychotic and/or antidepressant acitivity.

**9.** A compound of the formula II

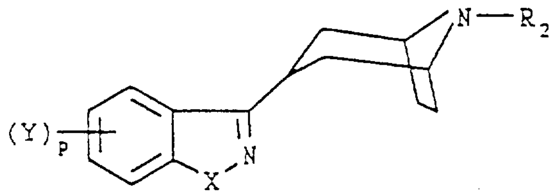

. (II)

wherein $R_2$ is hydrogen or loweralkyl and X, Y and p are as defined in claim 1.

## Patentansprüche

### Patentansprüche für folgende Vertragsstaaten : AT, BE, CH, LI, DE, DK, FR, GB, IT, LU, NL, PT, SE

**1.** Verbindung der Formel

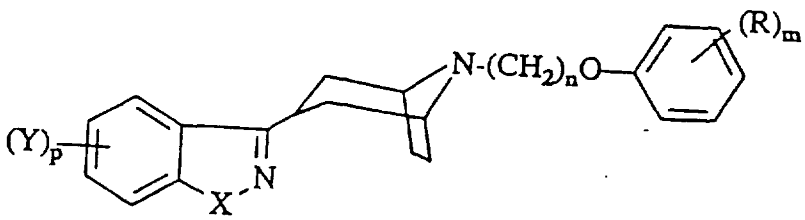

in welcher

X für -O-, -S- oder -NH- steht;
Y Wasserstoff, Halogen oder Niederalkoxy bedeutet;
p für 1 oder 2 steht;
n für 2, 3 oder 4 steht;
R Wasserstoff, Niederalkyl, Niederalkoxy, Hydroxy, Halogen, Arnino, Niederalkylamino, Nitro, Niederalkylthio, Trifluormethoxy, Cyano, Trifluormethyl,

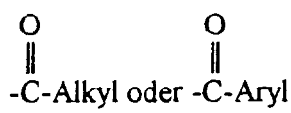

darstellt, wobei Aryl

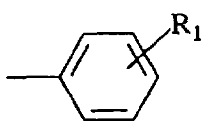

bedeutet;
$R_1$ Wasserstoff, Niederalkyl, Niederalkoxy, Halogen, Hydroxy, Carboxyl, Niederalkylamino, Nitro, Niederalkylthio, Cyano oder Trifluormethyl ist;
m 1 oder 2 bedeutet;

oder eine Verbindung der vorstehenden Formel, in welcher R für 3-Methoxy und 1-Acetamid, n für 3, X für -O- und Y für 6-Fluor steht;oder ein pharmazeutisch veträgliches Säureadditionssalz davon oder gegebenenfalls die geometrischen und optischen Isomere und racemischen Gemische davon.

**2.** Verbindung gemäß Anspruch 1, in welcher R für Niederalkoxy oder

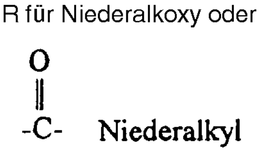

steht, Y Wasserstoff oder Halogen darstellt, p für 1 und m für 2 steht.

3. Verbindung gemäß Anspruch 1, in welcher X für O oder S steht, n für 3 und Y für Wasserstoff oder Fluor steht.

4. Verbindung gemäß Anspruch 1, in welcher X für O und R für $OCH_3$ oder

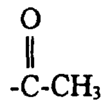

steht.

5. Verbindung gemäß Anspruch 1, bei der es sich um [4-[2-[3-[1,2-Benzisoxazol-3-yl]-8-azabicyclo-[3.2.1]-oktan-8-yl] ethoxy]-3-methoxyphenyl]ethanon oder ein pharmazeutisch verträgliches Säureadditionssalz davon handelt.

6. Verbindung gemäß Anspruch 1, bei der es sich um [4-[4-[3-[1H-Indazol-3-yl]-8-azabicyclo-[3.2.1]-oktan-8-yl] butoxy]-3-methoxyphenyl]ethanon oder ein pharmazeutisch verträgliches Säureadditionssalz davon handelt.

7. Verbindung gemäß Anspruch 1, bei der es sich um [4-[4-[3-[6-Fluor-1H-indazol-3-yl]-8-azabicyclo-[3.2.1]-oktan-8-yl]butoxy]-3-methoxyphenyl]ethanon oder ein pharmazeutisch verträgliches Säureadditionssalz davon handelt.

8. Pharmazeutische Zusammensetzung, die als Wirkstoffeine Verbindung gemäß Anspruch 1 sowie eine geeignete Trägersubstanz dafür enthält.

9. Verwendung einer Verbindung gemäß Anspruch 1 zur Herstellung eines Arzneimittels mit antipsychotischer und/oder antidepressiver Wirksamkeit.

10. Verfahren zur Herstellung einer Verbindung gemäß Anspruch 1, das die Umsetzung einer Verbindung der Formel XI, XVI oder XVIII

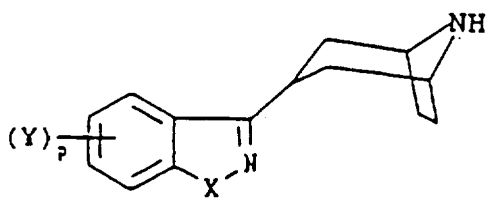

XI, XVI, XVIII

in welcher X, Y und p die vorstehend angewiesene Bedeutung zukommt, mit einer Verbindung der Formel XII

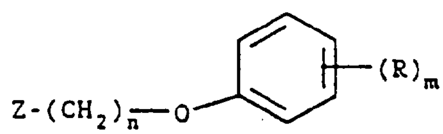

XII

in welcher Z für Chlor oder Brom steht, n für 2, 3 oder 4 steht und R und m die vorstehend angewiesene Bedeutung zukommt, zur Herstellung einer Verbindung der Formel I umfaßt, in welcher X, Y, p, n, m und R die vorstehend angewiesene Bedeutung zukommt.

11. Verbindung der Formel II

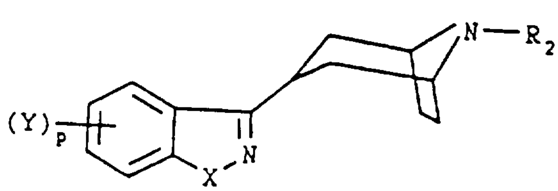

II

in welcher $R_2$ für Wasserstoff oder Niederalkyl steht und X, Y und P wie in Anspruch 1 angegeben definiert sind.

**Patentansprüche für folgende Vertragsstaaten : ES, GR**

1. Verfahren zur Herstellung einer Verbindung der Formel I

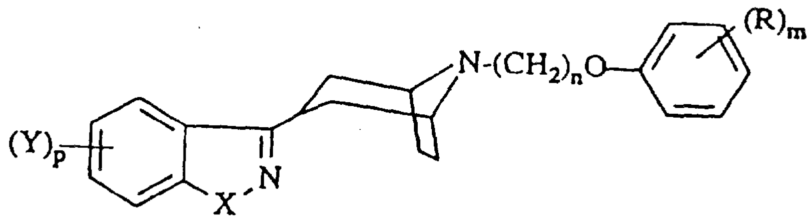

I

in welcher

- X für -O-, -S- oder -NH- steht;
- Y Wasserstoff, Halogen oder Niederalkoxy bedeutet;
- p für 1 oder 2 steht;
- n für 2, 3 oder 4 steht;
- R Wasserstoff, Niederalkyl, Niederalkoxy, Hydroxy, Halogen, Amino, Niederalkylamino, Nitro, Niederalkylthio, Trifluormethoxy, Cyano, Trifluormethyl,

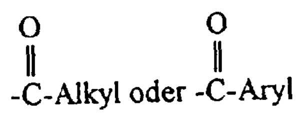

darstellt, wobei Aryl

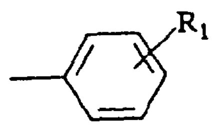

bedeutet;

- $R_1$ Wasserstoff, Niederalkyl, Niederalkoxy, Halogen, Hydroxy, Carboxyl, Niederalkylamino, Nitro, Niederalkylthio, Cyano oder Trifluormethyl ist;
- m 1 oder 2 bedeutet;

oder einer Verbindung der vorstehenden Formel, in welcher R für 3-Methoxy und 1-Acetamid, n für 3, X für -O- und Y für 6-Fluor steht; oder eines pharmazeutisch veträglichen Säureadditionssalzes davon oder gegebenenfalls der geometrischen und optischen Isomere und racemischen Gemische davon,
das die Umsetzung einer Verbindung der Formel XI, XVI oder XVIII

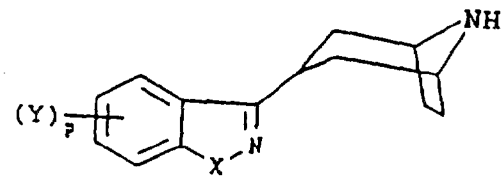

XI, XVI, XVII

in welcher X, Y und p die vorstehend angewiesene Bedeutung zukommt, mit einer Verbindung der Formel XII

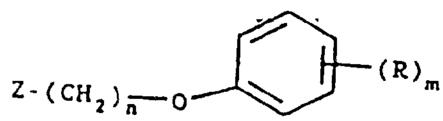

XII

in welcher Z für Chlor oder Brom steht, n für 2, 3 oder 4 steht und R und m die vorstehend angewiesene Bedeutung zukommt, zur Herstellung einer Verbindung der Formel I umfaßt, in welcher X, Y, p, n, m und R die vorstehend angewiesene Bedeutung zukommt.

2. Verfahren gemäß Anspruch 1, wobei R für Niederalkoxy oder

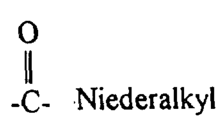

steht, Y Wasserstoff oder Halogen darstellt, p für 1 und m für 2 steht.

3. Verfahren gemäß Anspruch 1, wobei X für O oder S steht, n für 3 und Y für Wasserstoff oder Fluor steht.

**4.** Verfahren gemäß Anspruch 1, wobei X für O und R für $OCH_3$ oder

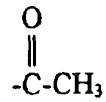

steht.

**5.** Verfahren gemäß Anspruch 1, bei dem [4-[2-[3-[1,2-Benzisoxazol-3-yl]-8-azabicyclo-[3.2.1]-oktan-8-yl]ethoxy]-3-methoxyphenyl]ethanon oder ein pharmazeutisch verträgliches Säureadditionssalz davon hergestellt wird.

**6.** Verfahren gemäß Anspruch 1, bei dem [4-[4-[3-[1H-Indazol-3-yl]-8-azabicyclo-[3.2.1]-oktan-8-yl]butoxy]-3-methoxyphenyl]ethanon oder ein pharmazeutisch verträgliches Säureadditionssalz davon hergestellt wird.

**7.** Verfahren gemäß Anspruch 1, bei dem [4-[4-[3-[6-Fluor-1H-indazo]-3-yl]-8-azabicyclo-[3.2.1]-oktan-8-yl]butoxy]-3-methoxyphenyl]ethanon oder ein pharmazeutisch verträgliches Säureadditionssalz davon hergestellt wird.

**8.** Verwendung einer Verbindung gemäß Anspruch 1 zur Herstellung eines Arzneimittels mit antipsychotischer und/oder antidepressiver Wirksamkeit.

**9.** Verbindung der Formel II

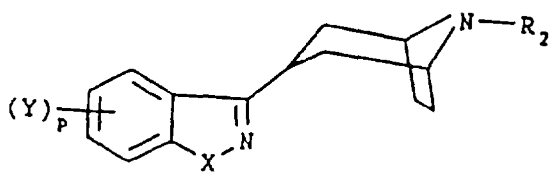

II

in welcher $R_2$ für Wasserstoff oder Niederalkyl steht und X, Y und P wie in Anspruch 1 angegeben dcfiniert sind.

## Revendications

**Revendications pour les Etats contractants suivants : AT, BE, CH, LI, DE, DK, FR, GB, IT, LU, NL, PT, SE**

**1.** Composé de formule I

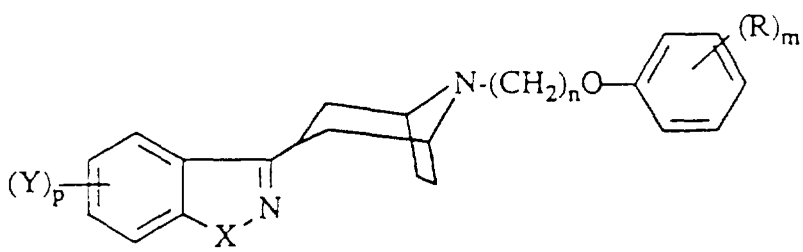

(I)

dans laquelle

X est -O-, -S- ou -NH-;
Y est hydrogène, halogène ou alcoxy inférieur;
p est 1 ou 2;
n est 2, 3 ou 4;
R est hydrogène, alkyle inférieur, alcoxy inférieur, hydroxy, halogène, amino, alkylamino inférieur, nitro, alkylthio inférieur, trifluorométhoxy, cyano, trifluorométhyle,

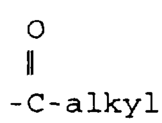 ou 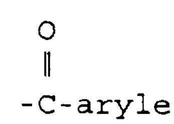

où aryle est

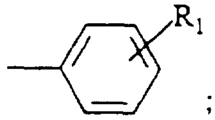 ;

$R_1$ est hydrogène, alkyle inférieur, alcoxy inférieur, halogène, hydroxy, carboxy, alkylamino inférieur, nitro, alkylthio inférieur, cyano ou trifluorométhyle;
m est 1 ou 2; ou un composé de même formule que ci-dessus dans laquelle R est 3-méthoxy et 1-acétamido, n est 3,
X est O et Y est 6-fluoro; ou un sel d'addition avec un acide pharmaceutiquement acceptable ou le cas échéant les isomères optiques et géométriques et les mélanges racémiques de ceux-ci.

2. Composé selon la revendication 1, dans lequel R est alcoxy inférieur ou

$$-\overset{\overset{\displaystyle O}{\|}}{C}-\text{alkyle}$$

inférieur, Y est hydrogène ou halogène, p est 1 et m est 2.

3. Composé selon la revendication 1, dans lequel X est O ou S, n est 3, Y est hydrogène ou fluoro.

4. Composé selon la revendication 1, dans lequel X est O et R est $OCH_3$ ou

$$-\overset{\overset{\displaystyle O}{\|}}{C}-CH_3.$$

5. Composé selon la revendication 1 qui est la [4-[2-[3-[1,2-benzisoxazol-3-yl]-8-azabicyclo [3.2.1] octan-8-yl]-éthoxy] -3-méthoxyphényl]éthanone ou un sel d'addition pharmaceutiquement acceptable de celui-ci.

6. Composé selon la revendication 1 qui est la [4-[4-[3-[1H-indazol-3-yl]-8-azabicyclo [3.2.1]octan-8-yl]-butoxy]-3-méthoxyphényl]éthanone ou un sel d'addition pharmaceutiquement acceptable de celui-ci.

7. Composé selon la revendication 1 qui est la [4-[4-[3-[6-fluoro-1H-indazol-3-yl]-8-azabicyclo [3.2.1] octan-8-yl]-butoxy]-3-méthoxyphényl]éthanone ou un sel d'addition pharmaceutiquement acceptable de celui-ci.

8. Composition pharmaceutique comprenant un composé selon la revendication 1 en tant que principe actif et un véhicule approprié.

9. Utilisation d'un composé selon la revendication 1 pour la préparation d'un médicament ayant une activité antipsychotique et/ou anti-dépressive.

10. Procédé pour la préparation d'un composé selon la revendication 1 qui comprend l'étape consistant à faire réagir un composé de formule XI, XVI ou XVIII

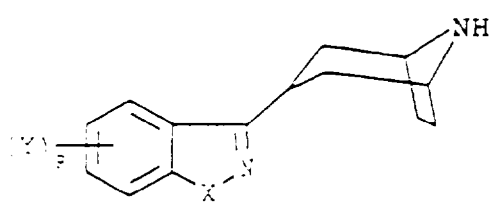 XI, XVI, XVIII

dans laquelle X, Y et p sont tels que définis à la revendication 1 avec un composé de formule XII

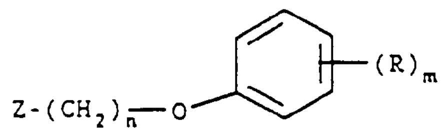 XII

dans laquelle Z est du chlore ou du brome et n est 2, 3 ou 4 et R et m sont tels que définis à la revendication 1 de façon à obtenir un composé de formule I dans laquelle X, Y, p, n, m et R sont tels que définis à la revendication 1.

**11.** Composé de formule II

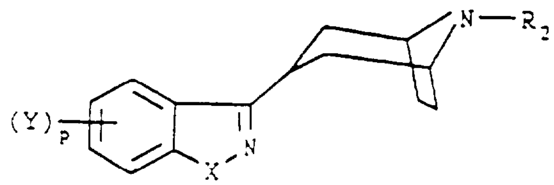 . (II)

dans laquelle $R_2$ est hydrogène ou alkyle inférieur et X, Y et p sont tels que définis dans la revendication 1.

**Revendications pour les Etats contractants suivants : ES, GR**

**1.** Procédé pour la préparation d'un composé de formule I

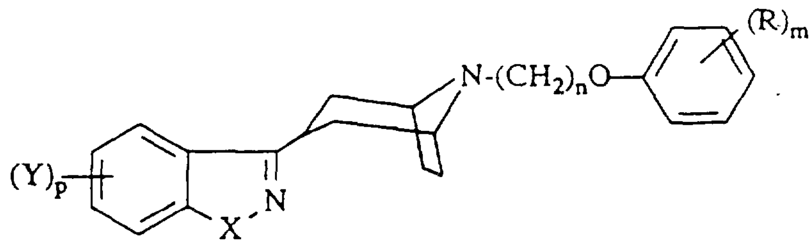

(I)

dans laquelle

X est -O-, -S- ou -NH-;
Y est hydrogène, halogène ou alcoxy inférieur;
p est 1 ou 2;
n est 2, 3 ou 4;
R est hydrogène, alkyle inférieur, alcoxy inférieur, hydroxy, halogène, amino, alkylamino inférieur, nitro, alkylthio inférieur, trifluorométhoxy, cyano, trifluorométhyle,

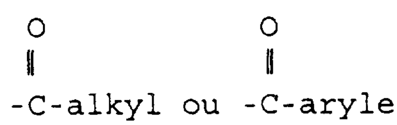

où aryle est

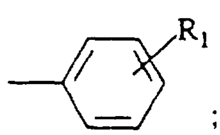 ;

$R_1$ est hydrogène, alkyle inférieur, alcoxy inférieur, halogène, hydroxy, carboxy, alkylamino inférieur, nitro, alkylthio inférieur, cyano ou trifluorométhyle;
m est 1 ou 2; ou un composé de même formule que ci-dessus dans laquelle R est 3-méthoxy et 1-acétamido, n est 3,
X est O et Y est 6-fluoro; ou un sel d'addition avec un acide pharmaceutiquement acceptable ou le cas échéant les isomères optiques et géométriques et les mélanges racémiques de ceux-ci,
lequel procédé comprend les étapes consistant à faire réagir un composé de formule XI, XVI ou XVIII

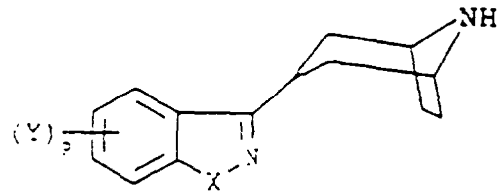

XI, XVI, XVIII

dans laquelle X, Y et p sont tels que définis ci-dessus avec un composé de formule XII

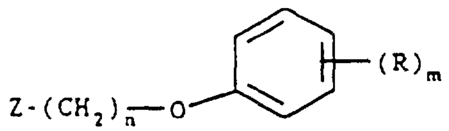

XII

XI, XVI, XVIII dans laquelle Z est du chlore ou du brome et n est 2, 3 ou 4 et R et m sont tels que définis ci-dessus de façon à obtenir un composé de formule I dans laquelle X, Y, p, n, m et R sont tels que définis ci-dessus.

**2.** Procédé selon la revendication 1, où R est alcoxy inférieur ou

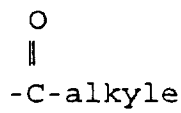

inférieur, Y est hydrogène ou halogène, p est 1 et m est 2.

**3.** Procédé selon la revendication 1, où X est O ou S, n est 3, Y est hydrogène ou fluoro.

**4.** Procédé selon la revendication 1, où X est O et R est $OCH_3$ ou

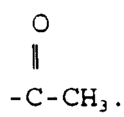

**5.** Procédé selon la revendication 1, pour la préparation de la [4-[2-[3-[1,2-benzisoxazol-3-yl]-8-azabicyclo [3.2.1] octan-8-yl]-éthoxy]-3-méthoxyphényl]éthanone ou un sel d'addition pharmaceutiquement acceptable de celui-ci.

**6.** Procédé selon la revendication 1, pour la préparation de la [4-[4-[3-[1H-indazol-3-yl]-8-azabicyclo [3.2.1]octan-8-yl]-butoxy]-3-méthoxyphényl]éthanone ou un sel d'addition pharmaceutiquement acceptable de celui-ci.

**7.** Procédé selon la revendication 1, pour la préparation de la [4-[4-[3-[6-fluoro-1H-indazol-3-yl]-8-azabicyclo [3.2.1] octan-8-yl]-butoxy]-3-méthoxyphényl]éthanone ou un sel d'addition pharmaceutiquement acceptable de celui-ci.

**8.** Utilisation d'un composé tel que défini à la revendication 1 pour la préparation d'un médicament ayant une activité antipsychotique et/ou anti-dépressive.

**9.** Composé de formule II

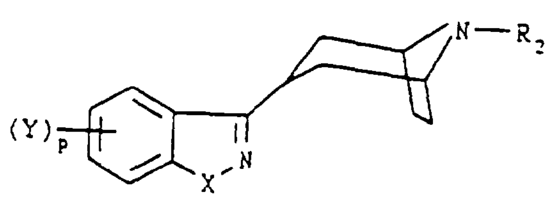

. (II)

dans laquelle $R_2$ est hydrogène ou alkyle inférieur et X, Y et p sont tels que définis dans la revendication 1.